(19) Europäisches Patentamt
European Patent Office
Office européen des brevets

(11) **EP 2 086 483 B1**

(12) **EUROPÄISCHE PATENTSCHRIFT**

(45) Veröffentlichungstag und Bekanntmachung des Hinweises auf die Patenterteilung:
**16.03.2016 Patentblatt 2016/11**

(21) Anmeldenummer: **07819765.4**

(22) Anmeldetag: **12.11.2007**

(51) Int Cl.:
***A61F 9/01*** *(2006.01)*

(86) Internationale Anmeldenummer:
**PCT/EP2007/009778**

(87) Internationale Veröffentlichungsnummer:
**WO 2008/055705 (15.05.2008 Gazette 2008/20)**

(54) **BEHANDLUNGSVORRICHTUNG ZUR OPERATIVEN FEHLSICHTIGKEITSKORREKTUR EINES AUGES UND VERFAHREN ZUM ERZEUGEN VON STEUERDATEN DAFÜR**

TREATMENT DEVICE FOR OPERATIVELY CORRECTING DEFECTIVE VISION OF AN EYE AND METHOD FOR PRODUCING CONTROL DATA

DISPOSITIF DE TRAITEMENT DE CORRECTION D'UN DÉFAUT VISUEL DE L'OEIL PAR OPÉRATION ET PROCÉDÉ DE PRODUCTION DE DONNÉES DE COMMANDE POUR CE DISPOSITIF

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **10.11.2006 DE 102006053119**
**10.11.2006 US 858201 P**

(43) Veröffentlichungstag der Anmeldung:
**12.08.2009 Patentblatt 2009/33**

(73) Patentinhaber: **Carl Zeiss Meditec AG**
**07745 Jena (DE)**

(72) Erfinder:
• **BISCHOFF, Mark**
**07749 Jena (DE)**
• **STOBRAWA, Gregor**
**07743 Jena (DE)**
• **STICKER, Markus**
**07749 Jena (DE)**

(74) Vertreter: **Patentanwälte Geyer, Fehners & Partner mbB**
**Perhamerstrasse 31**
**80687 München (DE)**

(56) Entgegenhaltungen:
**WO-A-2005/011547    US-A1- 2004 070 761**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung bezieht sich auf eine Behandlungsvorrichtung zur operativen Fehlsichtigkeitskorrektur eines Auges eines Patienten, die eine von einer Steuereinrichtung gesteuerte Lasereinrichtung aufweist, welche zur Isolierung eines in der Hornhaut liegende Volumens durch Einstrahlen von Laserstrahlung Hornhaut-Gewebe trennt, wobei die Steuereinrichtung durch Vorgabe von in der Hornhaut liegenden Zielpunkten die Lasereinrichtung zur Fokussierung der Laserstrahlung in die Hornhaut ansteuert.

Die Erfindung bezieht sich weiter auf einen Verfahren zum Erzeugen von Steuerdaten für eine Lasereinrichtung einer Behandlungsvorrichtung zur operativen Fehlsichtigkeitskorrektur eines Auges eines Patienten, welche zur Isolierung eines in der Hornhaut liegenden Volumens durch Einstrahlen fokussierter Laserstrahlung Hornhaut-Gewebe trennt, wobei die Steuerdaten im Betrieb der Behandlungsvorrichtung der Lasereinrichtung Zielpunkte für die fokussierte Laserstrahlung vorgeben, die in der Hornhaut liegen.

Der klassische Weg zur Korrektur der Fehlsichtigkeit des menschlichen Auges ist die Brille. Mittlerweile wird jedoch auch vermehrt refraktive Chirurgie eingesetzt, die durch Veränderung der Augenhornhaut eine Fehlsichtigkeitskorrektur bewirkt. Ziel der Operationsmethoden ist es dabei, die Hornhaut gezielt zu verändern, um so die Lichtbrechung zu beeinflussen. Hierfür sind unterschiedliche Operationsmethoden bekannt. Am verbreitesten ist gegenwärtig die sogenannte Laser-Insitu-Keratomileusis, die auch LASIK abgekürzt wird. Dabei wird zuerst eine Hornhautlamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. von Intralase Corp. Irvine, USA, vertrieben wird. Nachdem die Lamelle gelöst und zur Seite geklappt wurde, ist bei der LASIK-Operation die Anwendung eines Excimer-Lasers vorgesehen, der das derart freigelegte Hornhautgewebe durch Ablation abträgt. Nachdem auf diese Art und Weise in der Hornhaut liegendes Volumen verdampft wurde, wird die Hornhautlamelle wieder auf den ursprünglichen Platz zurückgeklappt.

Die Anwendung eines Laserkeratoms zum Freilegen der Lamelle ist vorteilhaft, da die Infektionsgefahr dadurch verringert und die Schnittqualität vergrößert ist. Insbesondere kann die Lamelle mit sehr viel konstanterer Dicke hergestellt werden. Auch ist der Schnitt potentiell glatter, was spätere optische Störungen durch diese auch nach der Operation verbleibende Grenzfläche mindert.

Bei der Erzeugung einer Schnittfläche in der Hornhaut durch Laserstrahlung laufen im Gewebe zeitlich hintereinander mehrere Prozesse ab, die durch die gepulste Laserstrahlung initiiert werden. Liegt die Leistungsdichte der Strahlung bei einem Puls über einem Schwellwert, kommt es zu einem optischen Durchbruch, der in der Hornhaut z.B. eine Plasmablase erzeugt. Die Plasmablase wächst nach Entstehen des optischen Durchbruchs durch sich ausdehnende Gase. Wird der optische Durchbruch nicht aufrechterhalten, so wird das in der Plasmablase erzeugte Gas vom umliegenden Gewebe aufgenommen, und die Blase verschwindet wieder. Es sind auch Gewebetrenneffekte möglich, die ohne Plasmablase wirken. Der Einfachheit halber werden all solche Prozesse hier unter dem Begriff "optischer Durchbruch" zusammengefaßt, d.h. dieser Begriff soll nicht nur den optischen Durchbruch sondern auch die daraus resultierenden Wirkungen in der Hornhaut einschließen.

Zur Gewebetrennung wird die Laserstrahlung gepulst angewendet, wobei die Pulslänge in der Regel unter 1 ps liegt. Dadurch wird die zur Auslösung des optischen Durchbruchs nötige Leistungsdichte für den jeweiligen Puls in einem engen räumlichen Gebiet erreicht. Die US 5984916 zeigt diesbezüglich deutlich, daß der räumliche Bereich des optischen Durchbruches (in diesem Fall der erzeugten Wechselwirkung) stark von der Pulsdauer abhängt. Eine hohe Fokussierung des Laserstrahls in Kombination mit den erwähnten kurzen Pulsen erlaubt es damit, den optischen Durchbruch punktgenau in der Hornhaut einzusetzen.

Zur Schnitterzeugung wird eine Serie optischer Durchbrüche an vorbestimmten Stellen so erzeugt, daß dadurch die Schnittfläche ausgebildet wird. Beim erwähnten Laserkeratom bildet die Schnittfläche die vor dem Einsatz der Laserablation abzuklappende Lamelle.

Die US 2004/0070761 A1 offenbart eines des eingangs genannten Laserkeratome, das mittels eines planen Kontaktglases die Augenhornhaut flachdrückt. Die WO 2005/011547 verwendet in der Lasechirurgie ein gekrümmtes Kontaktglas. Beide Druckschriften befassen sich mit der Korrektur von Verformungen der Hornhaut durch das Kontaktglas.

Die Präzision, minder die Schnittfläche erzeugt wird, ist natürlich für die optische Korrektur letztlich entscheidend. Dies gilt insbesondere für weitergebildete laserchirurgische Fehlsichtigkeitskorrekturverfahren, bei denen ein in der Hornhaut liegendes Volumen durch eine dreidimensionale Schnittfläche isoliert und so entfernbar gemacht wird. Anders als beim Laserkeratom ist dann die Lage der Schnittfläche direkt relevant für optische Korrektur. Bei der herkömmlichen LASIK-Methode ist dagegen ausschließlich die Präzision, mit der die Laserablation betrieben wird, für die Güte der optischen Korrektur wichtig, was man schon daran erkennt, daß die Erzeugung der Hornhautlamelle auch mit einem vergleichsweise grob arbeitenden mechanischen Messer möglich ist und auch in einer großen Vielzahl von Operationen praktiziert wird bzw. wurde.

Es ist deshalb Aufgabe der Erfindung, eine Behandlungsvorrichtung bzw. ein Verfahren der eingangs genannten Art so auszubilden, daß eine Schnittflächenerzeugung mit hoher Präzision erfolgen kann. Diese Aufgabe wird durch die in den Ansprüchen 1 und 8 definierte Erfindung gelöst.

Die Erfindung trägt der Tatsache Rechnung, daß optische Systeme in der Regel nicht perfekt sind. Ein Fehler bei der Fokussierung der Laserstrahlung in der Hornhaut wirkt sich auf die Schnittflächenerzeugung aus. Dies gilt insbesondere für einen Fehler der Fokuslage, durch den vermeintlich in einer Ebene positionierte Fokuslagen tatsächlich nicht in einer Ebene liegen, sondern in einer gekrümmten Fläche. Bei den bekannten Laserkeratomen spielt dieser Gesichtspunkt keine Rolle, da die Erzeugung des die Lamelle freilegenden Schnittes für die optische Qualität der Korrektur ohne Bedeutung ist. Da nun aber das zu isolierende und dann zu entnehmende Volumen gänzlich durch die Fokussierung der gepulsten Laserstrahlung definiert ist, sieht die erfindungsgemäße Behandlungsvorrichtung bzw. das erfindungsgemäße Verfahren vor, daß Fokuslagenfehler, die beim Fokussieren der Laserstrahlung, die vorzugsweise gepulst appliziert wird, eine Abweichung zwischen vorgegebener und tatsächlicher Lage der Zielpunkte bewirken, durch einen entsprechenden gegensinnigen Vorhalt berücksichtigt wird.

Der Vorhalt verursacht eine Vorverzerrung der Zielpunkte derart, daß in Kombination mit dem Fokuslagenfehler die Zielpunkte wieder an der Stelle liegen, die beim Einsatz der Behandlungsvorrichtung gewünscht sind.

Der Vorhalt kann zweckmäßigerweise durch eine Korrekturtabelle oder -funktion bestimmt werden, welche den Fokuslagenfehler abhängig von der Lage des jeweiligen Zielpunktes angibt. Diese Tabelle oder Funktion kann für den jeweiligen Gerätetyp einheitlich vorgeschrieben oder, was aus Gründen der Präzision bevorzugt ist, für das jeweilige Gerät individuell ermittelt werden, beispielsweise durch den Gerätehersteller oder nach Installation des Gerätes beim Anwender.

Mit vertretbarem Aufwand kann die Optik, die bei der Fokussierung der Laserstrahlung verwendet, so ausgebildet werden, daß der Fokuslagenfehler im wesentlichen ein axialer. Fokuslagenfehler ist und rotationssymmetrisch zur optischen Achse verläuft. Es ist für diese Variante dann bevorzugt, daß jeder Vorhalt eine Verschiebung des jeweiligen Zielpunktes parallel zur optischen Achse bewirkt und daß die Korrekturtabelle oder -funktion nur von einer axialen und einer radialen Koordinate abhängt, wenn die Zielpunkte in Zylinderkoordinaten beschrieben sind. Zweckmäßigerweise wird man den Ursprung der Zylinderkoordinate in den Durchstoßpunkt der optischen Achse durch die Hornhautvorderfläche legen.

Zur Ermittlung der Zielpunkte kann eine analytische Definition der Zielpunktmenge gewählt werden. Dies fällt besonders einfach, wenn eine Bahn festgelegt wird, entlang der der Fokus der Laserstrahlung zu verstellen ist, um die gewünschte Schnittfläche, welche dann das Volumen isoliert, auszubilden. Die Zielpunkte müssen lediglich aus den Punkten der Bahn ausgewählt werden. Da die Korrektur hinsichtlich des Fokuslagenfehlers in der Regel nicht als transzendente, analytische oder gar lineare Funktion vorliegen wird, sondern in den meisten Fällen bestenfalls eine Interpolation durch Polynome oder Splines möglich sein wird, ist es zu bevorzugen, die Zielpunkte vor der Korrektur aus der Bahn zu ermitteln. Dieses Vorgehen hat den Vorteil, daß ein einfacher, rechensparender Aufwand zur Ermittlung der Zielpunkte verwendet werden kann.

Die Einfachheit ist nochmals gesteigert, wenn die Bahn durch eine oder mehrere Funktionsgleichung(en) festgelegt ist, so daß die Auswertung der Funktionsgleichung(en) an verschiedenen Stützstellen die Zielpunkte liefert. Bis zu diesem Zeitpunkt ist dann eine transzendente Beschreibung der Strahlablenkung möglich, die naturgemäß einen sehr viel geringeren Rechenaufwand mit sich bringt, als wenn eine große Menge an Zielpunkten verwaltet wird. Die eigentlichen Zielkoordinaten liegen erst nach der Auswertung der Funktionsgleichung(en) vor. Sie stellen dann Steuerdaten dar, die beim Betrieb der Behandlungsvorrichtung Anwendung finden. Natürlich können diese Steuerdaten noch weiter bearbeitet werden, um geräteindividuelle Anpassungen, z.B. Ermittlung entsprechender Spannungspegel zu den Koordinaten, zuvor ermittelte Amplituden- oder Phasenverhalten von Galvanometerspiegeln etc., zu berücksichtigen.

Zur Schnittflächenerzeugung wird, wie bereits erwähnt, der Fokus der in der Regel gepulsten Laserstrahlung dreidimensional lageverstellt. Es wird deshalb in der Regel eine zweidimensionale Ablenkung der Laserstrahlung, z.B. durch Galvanometerspiegel, mit gleichzeitiger Fokusverstellung in der dritten Raumrichtung, z.B. durch ein Teleskop, kombiniert. Die Einstellung der Lage des Fokus ist natürlich für die Genauigkeit, mit der die Schnittfläche durch die Zielpunkte erzeugt werden kann, ausschlaggebend. Es hat sich hierzu als zweckmäßig erwiesen, ein Kontaktglas zu verwenden, das auf das Auge aufgesetzt wird und dieses fixiert. Ein solches Kontaktglas ist auch bei den eingangs erwähnten Laserkeratomen üblich. Das Kontaktglas hat regelmäßig auch die Funktion, der Hornhautvorderfläche eine bekannte Form zu verleihen. Bei den bekannten Laserkeratomen ist die Form eine Ebene, d.h. das Auge wird im Bereich der Hornhaut flachgedrückt. Da dies für den Patienten relativ unangenehm ist, wurde für Ansätze, die laserchirurgisch ein Volumen in der Hornhaut isolieren, bereits ein Kontaktglas mit gekrümmten dem Auge zugewandten Kontaktfläche beschrieben. Ein solches Kontaktglas verleiht der Hornhautvorderfläche dann eine bekannte Krümmung. Die Krümmung hat natürlich zwangsläufig eine Verformung der Augenhornhaut zur Folge. Diese Verformung ist um so größer, je stärker die Krümmung der Kontaktfläche von der tatsächlichen Hornhautkrümmung des Auges des Patienten abweicht. Um möglichst mit einer einheitlichen Kontaktflächenkrümmung arbeiten zu können, ist es deshalb vorteilhaft, die Hornhautverformung, die bei der Anwendung eines gekrümmten Kontaktglases auftritt, bei der Angabe der Zielpunkte zu berücksichtigen, damit unabhängig vom Grad der Verformung die Zielpunkte letztlich für das freie, d.h. nicht durch das Kontaktglas verformte Auge an den gewünschten Stellen liegen und dadurch letztlich eine gewünschte Grenzfläche für das zu entnehmende Volumen aufgebaut wird.

Die Berücksichtigung der Verformung der Hornhaut kann zum einen auf Basis der Zielpunkte, d.h. wenn die einzelnen Koordinaten der Zielpunkte festliegen, erfolgen. Zum anderen kann der Verformung auch durch eine entsprechende Transformation der Bahn, entlang der der Fokus verstellt werden soll, Rechnung getragen werden. Es ist deshalb bevorzugt, daß für die Behandlungsvorrichtung die Steuereinrichtung bei der Festlegung der Bahn oder der Zielpunkte eine Verformung der Hornhaut des Auges berücksichtigt, die während des Einstrahlens der gepulsten Laserstrahlung auftritt, insbesondere durch ein Kontaktglas begründet ist. Analoges gilt für die erfindungsgemäßen Verfahren.

Insbesondere wenn die Bahn durch transzendente Funktionsgleichungen angegeben ist, ist es rechentechnisch besonders günstig, zuerst die Bahn bezogen auf das unverformte Auge festzulegen und die Bahn dann zum Ausgleichen der Verformung der Hornhaut des Auges zu modifizieren. In einem letzten Schritt kann dann die Auswertung der Bahnfunktion(en) an bestimmten Stützstellen erfolgen, um die Koordinaten für die Zielpunkte zu ermitteln, die dann in einem letzten Schritt hinsichtlich der Fokuslagenfehler wie beschrieben korrigiert werden.

Eine besonders einfache rechnerische Behandlung erhält man, wenn die Zielpunkte entlang Bahnen angeordnet sind, die spiralförmig verlaufen. Beispielsweise kann die zu erzeugende Schnittfläche, welche das Volumen isoliert, in zwei Teilflächen aufgeteilt werden, eine anteriore und eine posteriore Teilfläche. Die Teilflächen können jeweils durch Verstellen des Fokus entlang einer spiralförmigen Bahn gebildet werden.

Das erfindungsgemäße Verfahren zum Vorbereiten der Steuerdaten kann ohne menschliche Mitwirkung ausgeführt werden. Insbesondere kann es von einem Computer durchgeführt werden, der aus entsprechenden Vorgaben, beispielsweise aus den funktionell definierten Bahnkurven die Steuerdaten ermittelt, indem die Stützstellenwahl passend zur Verstellgeschwindigkeit der das Zielsystem zur Applikation der Steuerdaten darstellenden Lasereinrichtung ermittelt. Insbesondere ist bei der Ermittlung der Steuerdaten die Mitwirkung eines Arztes in keiner Weise erforderlich, da mit der Ermittlung der Steuerdaten noch kein therapeutischer Eingriff verbunden ist. Dieser findet erst bei der Anwendung der zuvor ermittelten Steuerdaten statt.

Die Erfindung wird nachfolgend unter Bezugnahme auf die Zeichnungen beispielshalber noch näher erläutert. In den Zeichnungen zeigt:

Fig. 1 eine Schemadarstellung einer Behandlungsvorrichtung bzw. eines Behandlungsgerätes zur Fehlsichtigkeitskorrektur,
Fig. 1a eine Schemadarstellung hinsichtlich des Aufbaus des Behandlungsgerätes der Fig. 1,
Fig. 2 eine Prinzipdarstellung zur Einbringung gepulster Laserstrahlung in das Auge bei der Fehlsichtigkeitskorrektur mit dem Behandlungsgerät der Fig. 1,
Fig. 3 eine weitere Schemadarstellung des Behandlungsgerätes der Fig. 1,
Fig. 4 in Teilfiguren (a), (b) und (c) schematische Schnittdarstellungen zur Verdeutlichung des Korrekturbedarfes am menschlichen Auge bei Fehlsichtigkeit,
Fig. 5 eine schematische Schnittdarstellung durch die Augenhornhaut mit Darstellung eines zur Fehlsichtigkeitskorrektur zu entfernenden Volumens,
Fig. 6 ein Schnitt durch die Augenhornhaut nach Entfernung des Volumens der Fig. 5,
Fig. 7 eine Schnittdarstellung ähnlich der Fig. 5,
Fig. 8 eine schematische Schnittdarstellung durch die Augenhornhaut zur Veranschaulichung der Volumenentnahme,
Fig. 9 eine Draufsicht auf eine spiralförmige Bahnkurve, die zur Isolierung des Volumens der Fig. 5, 7 und 8 verwendet wird,
Fig. 10 eine vergrößerte Darstellung der Bahnkurve der Fig. 9,
Fig. 11 eine alternative Bahnkurve für die Korrektur auch zylindrischer Fehlsichtigkeiten,
Fig. 12 eine schematische Darstellung zur Erläuterung der Funktion eines Kontaktglases im Behandlungsgerät der Fig. 1,
Fig. 13 bis 15 schematische Darstellungen hinsichtlich der Wirkungen des Kontaktglases durch Verformung der Augenhornhaut,
Fig. 16 und 17 Schemadarstellung hinsichtlich der Approximation der das Volumen posterior begrenzenden Fläche im Falle auch zylindrischer Fehldichtigkeitskorrekturen,
Fig. 18 eine schematische Darstellung zu einer bei der Ermittlung von Steuerdaten für das Behandlungsgerät der Fig. 1 verwendeten Fokuslagenfehlerkorrektur und
Fig. 19 eine schematische Darstellung des Ablaufes bei der Vorbereitung und Durchführung einer Fehlsichtigkeitskorrektur.

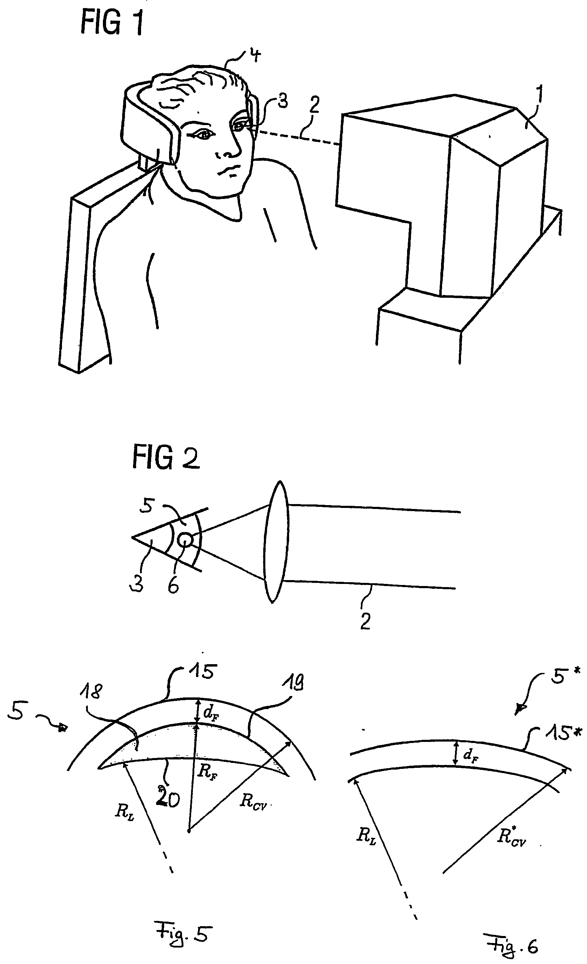

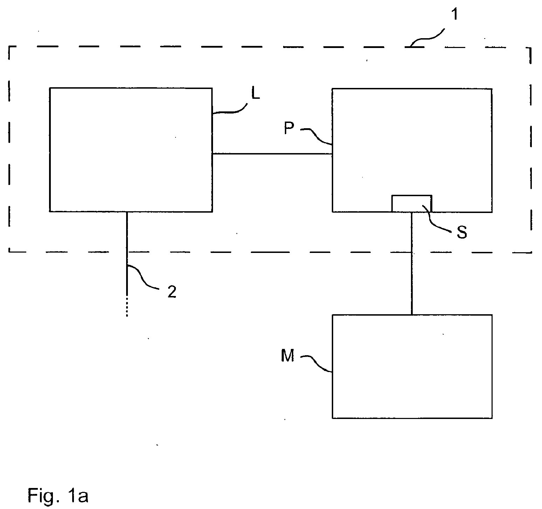

Figur 1 zeigt ein Behandlungsgerät 1 für ein augenchirurgisches Verfahren, das dem in der EP 1159986 A1 bzw. der US 5549632 beschriebenen ähnelt. Das Behandlungsgerät 1 bewirkt mittels ein Behandlungs-Laserstrahlung 2 eine Fehlsichtigkeitskorrektur an einem Auge 3 eines Patienten 4. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Abberationen höherer Ordnung umfassen. Die Behandlungs-Laserstrahlung 2 wird in der beschriebenen Ausführungsform als gepulster in das Auge 3 fokussierter Laserstrahl aufgebracht. Die Pulsdauer liegt dabei z.B. im Femtosekundenbereich, und die Laserstrahlung 2 wirkt mittels nicht-linearer optischer Effekte in der Hornhaut. Der Laserstrahl weist z.B. 50 bis 800 fs kurze Laserpulse (bevorzugt 100 - 400 fs) mit einer Pulswiederholfrequenz zwischen 10 und 500 kHz auf. Die Baugruppen des Gerätes 1 werden im beschriebenen Ausführungsbeispiel von einer integrierten Steuereinheit gesteuert, die aber natürlich auch eigenständig ausgebildet sein kann.

Vor dem Einsatz des Behandlungsgerätes wird die Fehlsichtigkeit des Auges 3 mit einer oder mehreren Meßeinrichtungen vermessen.

Figur 1a zeigt schematisch das Behandlungsgerät 1. Es weist in dieser Variante mindestens zwei Einrichtungen oder Module auf. Eine Lasereinrichtung L gibt den Laserstrahl 2 auf das Auge 3 ab. Der Betrieb der Lasereinrichtung L erfolgt dabei vollautomatisch, d.h. die Lasereinrichtung L startet auf ein entsprechendes Startsignal hin die Ablenkung des Laserstrahls 2 und erzeugt dabei Schnittflächen, die auf noch zu beschreibende Art und Weise aufgebaut sind und ein Volumen in der Augenhornhaut isolieren. Die den Betrieb erforderlichen Steuerdaten empfängt die Lasereinrichtung L zuvor von einer Planungseinrichtung P als Steuerdatensatz über nicht näher bezeichnete Steuerleitungen. Die Übertragung findet vor dem Betrieb der Lasereinrichtung L statt. Natürlich kann Kommunikation auch drahtlos erfolgen. Alternativ zu einer direkten Kommunikation ist es auch möglich, die Planungseinheit P räumlich getrennt von der Lasereinheit L anzuordnen und einen entsprechenden Datenübertragungskanal vorzusehen.

Vorzugsweise wird der Steuerdatensatz zum Behandlungsgerät 1 übertragen und weiter vorzugsweise ist ein Betrieb der Lasereinrichtung L gesperrt, bis an der Lasereinrichtung L ein gültiger Steuerdatensatz vorliegt. Ein gültiger Steuerdatensatz kann ein Steuerdatensatz sein, der prinzipiell zur Verwendung mit der Lasereinrichtung L der Behandlungsvorrichtung 1 geeignet ist. Zusätzlich kann die Gültigkeit aber auch daran geknüpft werden, daß weitere Prüfungen bestanden werden, beispielsweise ob im Steuerdatensatz zusätzlich niedergelegte Angaben über das Behandlungsgerät 1, z. B. eine Geräteseriennummer, oder den Patienten, z. B. eine Patientenidentifikationsnummer, mit anderen Angaben übereinstimmen, die beispielsweise an der Behandlungsvorrichtung ausgelesen oder separat eingegeben wurden, sobald der Patient in der korrekten Stellung für den Betrieb der Lasereinrichtung L ist.

Die Planungseinheit P erzeugt den Steuerdatensatz, der der Lasereinheit L zur Ausführung der Operation zur Verfügung gestellt wird, aus Meßdaten und Fehlsichtigkeitsdaten, die für das zu behandelnde Auge ermittelt wurden. Sie werden der Planungseinheit P über eine Schnittstelle S zugeführt und stammen im dargestellten Ausführungsbeispiel aus einer Meßeinrichtung M, die das Auge des Patienten 4 zuvor vermessen hat. Natürlich kann die Meßeinrichtung M auf beliebige Art und Weise die entsprechenden Meß- und Fehlsichtigkeitsdaten an die Planungseinrichtung P übermitteln.

Die Übertragung kann mittels Speicherchips (z.B. per USB oder memory stick), Magnetspeichern (z.B. Disketten), per Funk (z.B. WLAN, UMTS, Bluetooth) oder drahtgebunden (Z.B. USB, Firewire, RS232, CAN-Bus, Ethernet etc.) erfolgen. Gleiches gilt natürlich hinsichtlich der Datenübertragung zwischen Planungseinrichtung P und Lasereinrichtung L.

Eine direkte Funk- oder Draht-Verbindung der Meßeinrichtung M mit der Behandlungseinrichtung 1 hinsichtlich der Datenübertragung, die in einer Variante verwendet werden kann, hat den Vorteil, daß die Verwendung falscher Meß- und Fehlsichtigkeitsdaten mit größtmöglicher Sicherheit ausgeschlossen ist. Dies gilt insbesondere dann, wenn die Überführung des Patienten von der Meßeinrichtung M bzw. den Meßeinrichtungen zur Lasereinrichtung L mittels einer (in der Figur nicht dargestellten) Lagerungseinrichtung erfolgt, die mit der Meßeinrichtung M bzw. der Lasereinrichtung L so zusammenwirkt, daß die jeweiligen Einrichtungen erkennen, ob der Patient 4 in der jeweiligen Position zum Vermessen bzw. Einbringen der Laserstrahlung 2 ist. Mit einem Verbringen des Patienten 4 von der Meßeinrichtung M zur Lasereinrichtung L kann dabei zugleich auch die Übertragung der Meß- und Fehlsichtigkeitsdaten an die Behandlungsvorrichtung 1 erfolgen.

Es ist vorzugsweise durch geeignete Mittel sichergestellt, daß die Planungseinrichtung P immer den zum Patienten 4 gehörenden Steuerdatensatz erzeugt, und eine irrtümliche Verwendung eines falschen Steuerdatensatzes für einen Patienten 4 ist so gut wie ausgeschlossen.

Die Wirkungsweise des Laserstrahls 2 ist in Figur 2 schematisch angedeutet. Der Behandlungs-Laserstrahl 2 wird mittels einer nicht näher bezeichneten Optik in die Hornhaut 5 des Auges 6 fokussiert. Dadurch entsteht in der Hornhaut 5 ein Fokus, der einen Spot 6 überdeckt und in dem die Laserstrahlungsenergiedichte so hoch ist, daß in Kombination mit der Pulslänge ein nicht-linearer Effekt im Auge auftritt. Beispielsweise kann jeder Puls der gepulsten Laserstrahlung 2 am jeweiligen Spot 6 einen optischen Durchbruch in der Augenhornhaut 5 erzeugen, welcher wiederum eine in Figur 2 schematisch angedeutete Plasmablase initiiert. Dadurch wird dieses Laserpulses in der Hornhaut 5 Gewebe getrennt. Bei Entstehung einer Plasmablase umfaßt die Gewebeschichttrennung ein größeres Gebiet, als den Spot 6, welchen der Fokus der Laserstrahlung 2 überdeckt, obwohl die Bedingungen zur Erzeugung des Durchbruches nur im Fokus erreicht werden. Damit von jedem Laserpuls ein optischer Durchbruch erzeugt wird, muß die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt.

Alternativ kann ein gewebetrennender Effekt durch die gepulste Laserstrahlung auch dadurch erzeugt werden, daß mehrere Laserstrahlungspulse im einen Bereich abgegeben werden, wobei sich für mehrere Laserstrahlungspulse die Spots 6 überlappen. Es wirken dann mehrere Laserstrahlungspulse zusammen, um einen gewebetrennenden Effekt zu erreichen.

Die Art der Gewebetrennung, die das Behandlungsgerät 1 einsetzt, ist jedoch für die nachfolgende Beschreibung nicht weiter relevant, wesentlich ist lediglich, daß dazu gepulste Behandlungs-Laserstrahlung 2 verwendet wird. Beispielsweise kann ein Behandlungsgerät 1 verwendet werden, wie sie in der WO 2004/032810 A2 beschrieben ist. Wesentlich ist weiter, daß eine Vielzahl von Laserpulsfoki im Gewebe eine Schnittfläche ausbildet, deren Form vom Muster abhängt, mit dem die Laserpulsfoki im Gewebe angeordnet sind/werden. Das Muster gibt Zielpunkte für die Fokuslage vor, an denen ein oder mehrere Laserpuls(e) abgegeben wird(werden), und definiert die Form und Lage der Schnittfläche. Für die nachfolgend erläuterten Verfahren und Vorrichtungen ist das Muster der Zielpunkte von Bedeutung und wird noch näher beschrieben werden.

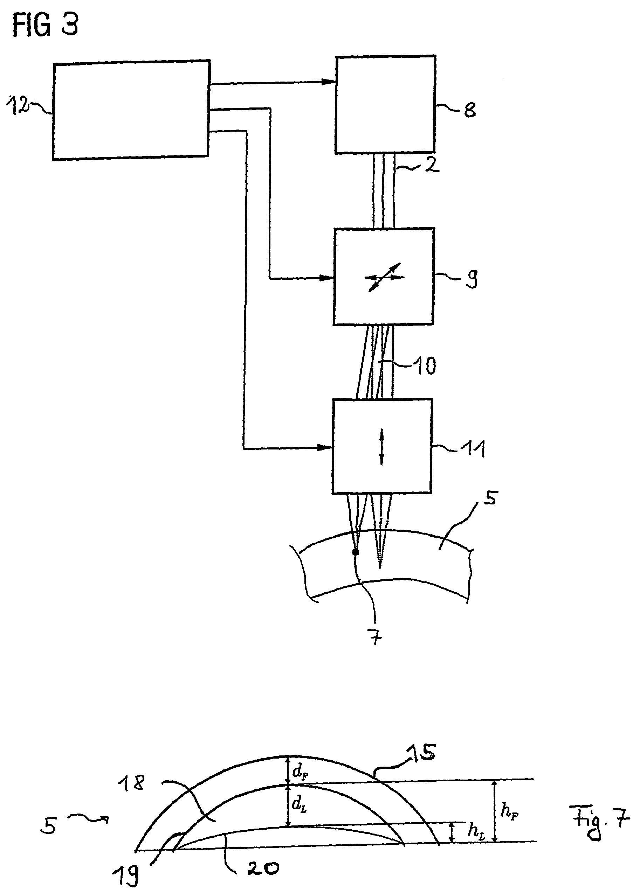

Um nun eine Fehlsichtigkeitskorrektur auszuführen, wird mittels der gepulsten Laserstrahlung aus einem Gebiet innerhalb der Hornhaut 5 Material entfernt, indem dort Gewebeschichten getrennt werden, die das Material isolieren und dann eine Materialentnahme ermöglichen. Die Materialentfernung bewirkt eine Volumenänderung in der Hornhaut, welche eine Änderung der optischen Abbildungswirkung der Hornhaut 5 zur Folge hat, die genau so bemessen ist, daß damit die zuvor ermittelte Fehlsichtigkeit möglichst korrigiert ist/wird. Zur Isolierung des zu entfernenden Volumens wird der Fokus der Laserstrahlung 2 auf Zielpunkte in der Hornhaut 5 gerichtet, in der Regel in einem Bereich, der unterhalb des Epithels und der Bowman'schen Membran sowie oberhalb der Decemetschen Membran und des Endothels liegt. Das Behandlungsgerät 1 weist dazu einen Mechanismus zum Verstellen der Lage des Fokus der Laserstrahlung 2 in der Hornhaut 5 auf. Dies ist schematisch in Figur 3 gezeigt.

In Figur 3 sind Elemente des Behandlungsgeräts 1 nur insoweit eingetragen, als sie zum Verständnis der Fokusverstellung erforderlich sind. Die Laserstrahlung 2 wird, wie bereits erwähnt, in einem Fokus 7 in der Hornhaut 5 gebündelt, und die Lage des Fokus 7 in der Hornhaut wird verstellt, so daß zur Schnittflächenerzeugung an verschiedenen Stellen fokussiert Energie aus Laserstrahlungspulsen in das Gewebe der Hornhaut 3 eingetragen wird. Die Laserstrahlung 2 wird von einem Laser 8 als gepulste Strahlung bereitgestellt. Ein xy-Scanner 9, der in einer Variante durch zwei im wesentlichen orthogonal ablenkende Galvanometerspiegel realisiert ist, lenkt den vom Laser 8 kommenden Laserstrahl zweidimensional ab, so daß nach dem xy-Scanner 9 ein abgelenkter Laserstrahl 10 vorliegt. Der xy-Scanner 9 bewirkt somit eine Verstellung der Lage des Fokus 7 im wesentlichen senkrecht zur Haupteinfallsrichtung der Laserstrahlung 2 in die Hornhaut 5. Zur Verstellung der Tiefenlage ist neben dem xy-Scanner 9 ein z-Scanner 11 vorgesehen, der beispielsweise als verstellbares Teleskop ausgebildet ist. Der z-Scanner 11 sorgt dafür, daß die z-Position der Lage des Fokus 7, d.h. dessen Position auf der optischen Achse des Einfalls verändert wird. Der z-Scanner 11 kann dem xy-Scanner 9 nach- oder vorgeordnet sein. Die nachfolgend mit x, y, z bezeichneten Koordinaten beziehen sich also auf die Ablenkung der Lage des Fokus 7.

Für das Funktionsprinzip des Behandlungsgerätes 1 ist die Zuordnung der einzelnen Koordinaten zu den Raumrichtungen nicht wesentlich, der einfacheren Beschreibung halber ist jedoch nachfolgend mit z immer die Koordinate entlang der optischen Achse des Einfalls der Laserstrahlung 2 bezeichnet, und x sowie y bezeichnen zwei zueinander orthogonale Koordinaten in einer Ebene senkrecht zur Einfallsrichtung des Laserstrahls. Dem Fachmann ist natürlich bekannt, daß eine dreidimensionale Beschreibung der Lage des Fokus 7 in der Hornhaut 5 auch durch andere Koordinatensysteme erfolgen kann, insbesondere muß es sich nicht um ein rechtwinkliges Koordinatensystem handeln. Daß der xy-Scanner 9 um zueinander rechtwinklige Achsen ablenkt ist also nicht zwingend, vielmehr kann jeder Scanner verwendet werden, der in der Lage ist, den Fokus 7 in einer Ebene zu verstellen, in der die Einfallsachse der optischen Strahlung nicht liegt. Somit sind auch schiefwinklige Koordinatensysteme möglich.

Weiter können auch nicht-kartesische Koordinatensysteme zur Beschreibung bzw. Steuerung der Lage des Fokus 7 verwendet werden, wie dies nachfolgend auch noch erläutert wird. Beispiele für solche Koordinatensysteme sind Kugelkoordinaten (auch als sphärische Koordinaten bezeichnet) sowie zylindrische Koordinaten.

Zur Steuerung der Lage des Fokus 7 werden der xy-Scanner 9 sowie der z-Scanner 11, die gemeinsam ein konkretes Beispiel einer dreidimensionalen Fokusverstelleinrichtung realisieren, von einem Steuergerät 12 über nicht näher bezeichnete Leitungen angesteuert. Gleiches gilt für den Laser 8. Das Steuergerät 3 sorgt für einen geeignet synchronen Betrieb des Lasers 8 sowie der dreidimensionalen Fokusverstelleinrichtung, exemplarisch realisiert durch den xy-Scanner 9 sowie den z-Scanner 11, so daß die Lage des Fokus 7 in der Hornhaut 5 so verstellt wird, daß letztendlich ein Material bestimmten Volumens isoliert wird, wobei die spätere Volumenentfernung eine gewünschte Fehlsichtigkeitskorrektur bewirkt.

Das Steuergerät 12 arbeitet nach vorgegebenen Steuerdaten, welche die Zielpunkte für die Fokusverstellung vorgeben. Die Steuerdaten sind in der Regel in einem Steuerdatensatz zusammengefaßt. Dieser gibt in einer Ausführungsform die Koordinaten der Zielpunkte als Muster vor, wobei die Reihenfolge der Zielpunkte im Steuerdatensatz die Aneinanderreihung der Fokuslagen und damit letztlich eine Bahnkurve (hier auch verkürzt als Bahn bezeichnet) festlegt. Der Steuerdatensatz enthält in einer Ausführungsform die Zielpunkte als konkrete Stellwerte für den Fokuslagenverstellmechanismus, z.B. für den xy-Scanner 9 und den z-Scanner 11. Zur Vorbereitung des augenchirurgischen Verfahrens, also bevor das eigentliche Operationsverfahren ausgeführt werden kann, werden die Zielpunkte und vorzugsweise auch deren Reihenfolge im Muster bestimmt. Es muß eine Vorplanung des operativen Eingriffes dahingehend erfolgen, daß die Steuerdaten für das Behandlungsgerät 1 ermittelt werden, deren Anwendung dann eine für den Patienten 4 optimale Fehlsichtigkeitskorrektur erreicht.

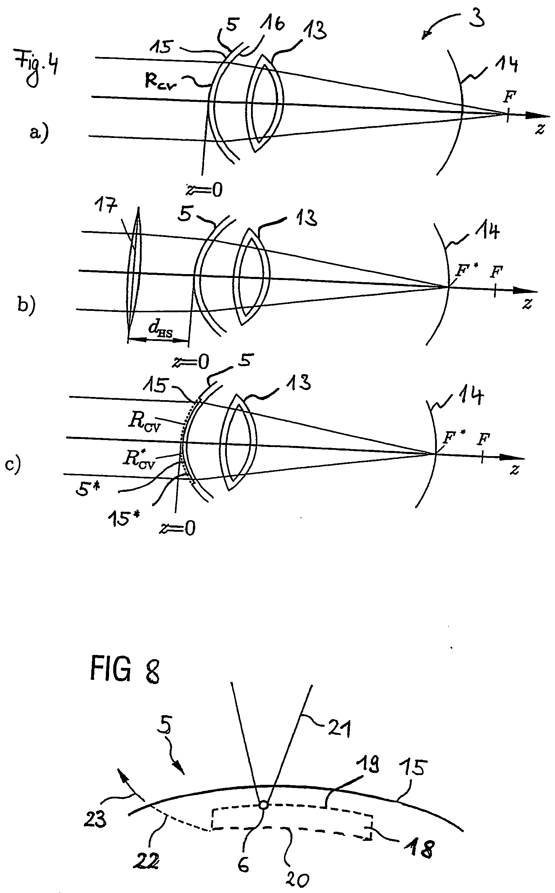

Zuerst gilt es das aus in der der Hornhaut 5 zu isolierende und später zu entfernende Volumen festzulegen. Wie bereits anhand Fig. 1a geschildert bedarf es dazu einer Feststellung des Korrekturbedarfs. Figur 4 zeigt in Teilfiguren a), b) und c) die optischen Verhältnisse am Auge 3 des Patienten 4. Ohne Fehlsichtigkeitskorrektur liegt die in Teilfigur a) gezeigte Situation vor.

Die Hornhaut 5 bewirkt zusammen mit der Augenlinse 13 eine Fokussierung eines im Unendlichen liegenden Gegenstandes in einen Fokus F, der auf der z-Achse hinter der Netzhaut 14 liegt. Die abbildende Wirkung rührt dabei zum einen von der bei nichtakkomodiertem Auge entspannten Augenlinse 13 sowie zum anderen von der Augenhornhaut 5 her, die im wesentlichen durch eine Hornhautvorderfläche 15 sowie eine Hornhautrückseite 16 definiert ist und aufgrund ihrer Krümmung ebenfalls eine abbildende Wirkung hat. Die optische Wirkung der Hornhaut 5 ist durch den Krümmungsradius $R_{CV}$ der Hornhautvorderfläche bedingt. Teilfigur a) stellt die Fehlsichtigkeit nur exemplarisch dar, real können die oben erwähnten komplexeren Fehlsichtigkeiten vorliegen. Für sie gilt die nachfolgenden Beschreibung jedoch ebenfalls, allerdings können die angegebenen Gleichungen dann mitunter eine zusätzliche Winkelabhängigkeit beinhalten, auch wenn darauf nicht ausdrücklich hingewiesen wird.

Zur Fehlsichtigkeitskorrektur wird bekannter Weise, wie in Teilfigur b) der Figur 4 dargestellt, eine Vorsatz-Linse 17 in Form einer Brille im Abstand $d_{HS}$ vom Scheitelpunkt der Hornhaut 5 vor das Auge 3 gesetzt. Die Linse 17 der Brille ist in ihrer Brechkraft $B_{BR}$ so angepaßt, daß sie den Fernpunkt des gesamten Systems, d.h. aus Brille und Auge, vom Fokuspunkt F zum korrigierten Fokuspunkt F* verschiebt, der auf der Netzhaut 14 liegt.

Hinsichtlich der in dieser Beschreibung verwendeten Nomenklatur sei angemerkt, daß durch die Anfügung eines Sterns an Größen verdeutlicht wird, daß es sich um Größen handelt, die nach einer Korrektur erhalten werden. Der Fokus F* ist also derjenige Fokus, der nach der optischen Korrektur vorliegt, die in der Teilfigur b) der Figur 4 durch die Linse 17 der Brille erreicht wird.

Unter der gerechtfertigten Annahme, daß eine Dickenänderung der Hornhaut 5 im wesentlichen den Krümmungsradius der Luft zugewandten Hornhaut-Vorderseite 15 modifiziert, nicht aber den Krümmungsradius der dem Augeninneren zuliegenden Hornhautrückseite 16, wird durch die Volumenentfernung der Krümmungsradius $R_{CV}$ der Hornhautvorderseite 15 modifiziert. Die um das Volumen verminderte Hornhaut 5 hat eine derart geänderte Abbildungswirkung, daß der dann korrigierte Fokus F* auf der Netzhaut 14 liegt. Nach der Korrektur liegt eine veränderte Hornhautvorderfläche 15* vor, und es ist eine Fehlsichtigkeitskorrektur auch ohne Brille erreicht.

Zur Bestimmung des Musters der Zielpunkte wird deshalb die zu erreichende Krümmung der modifizierten Hornhautvorderfläche 15* ermittelt. Dabei ist Ausgangspunkt die Brechkraft der Linse 17 der Brille, da die Ermittlung der entsprechenden Parameter ein Standardverfahren in der Augenoptik ist. Für die Brechkraft $B_{BR}(\varphi)$ der Linse 17 der Brille gilt folgende Formel:

$$(1) \qquad B_{BR}(\varphi) = Sph + Cyl \cdot \sin^2(\varphi - \theta).$$

In dieser Gleichung bezeichnen Sph und Cyl die zu realisierenden Korrekturwerte spärischen bzw. astigmatischen Brechungsfehler und $\theta$ die Lage der Zylinderachse der zylindrischen (astigmatischen) Fehlsichtigkeit, wie sie dem Fachmann in der Optometrie bekannt sind. Der Parameter $\varphi$ schließlich bezieht sich auf ein Zylinderkoordinatensystem des Auges und wird auf das Auge schauend entgegen dem Uhrzeigersinn gezählt, wie es in der Augenoptik üblich ist. Mit dem Wert $B_{BR}$ wird nun die Krümmung der modifizierten Hornhautvorderfläche 15* wie folgt eingestellt:

$$(2) \qquad R_{CV}^* = 1 / ( ( 1/R_{CV}) + B_{BR} / ( ( n_c\text{-}1) (1 - d_{HS} \cdot B_{BR}))) + F$$

In Gleichung (2) bezeichnet $n_c$ die Brechkraft des Materials der Hornhaut. Der entsprechende Wert liegt üblicherweise bei 1,376; $d_{HS}$ bezeichnet den Abstand, in dem eine Brille mit der Brechkraft $B_{BR}$ vom Hornhautscheitel liegen muß, um die gewünschte Fehlsichtigkeitskorrektur mittels Brille zu erzeugen; $B_{BR}$ bezeichnet die zuvor erwähnte Brechkraft der Brille gemäß Gleichung (1). Die Angabe für die Brechkraft $B_{BR}$ kann auch Fehlsichtigkeiten erfassen, die über eine normale sphärische oder zylindrische Korrektur hinausgehen. $B_{BR}$ (und damit automatisch auch $R_{CV}^*$) haben dann zusätzliche Koordinatenabhängigkeiten.

Der Faktor F drückt die optische Wirkung der Dickenänderung der Hornhaut aus und kann in erster Näherung als konstanter Faktor angesehen werden. Für eine hochgenaue Korrektur kann der Faktor gemäß folgender Gleichung errechnet werden:

$$(3) \qquad F = (1 - 1/n_c) \cdot (d_C{}^* - d_C).$$

$d_C$ bzw. $d_C{}^*$ ist dabei die Hornhautdicke vor bzw. nach der optischen Korrektur. Für eine genaue Bestimmung erfolgt eine Berechnung von $R_{CV}{}^*$ iterativ, indem bei der i-ten Berechnung aus der Differenz $(R_{CV}{}^* - R_{CV})$ auf die Größe $(d_C{}^* - d_C)$ geschlossen wird und das entsprechende daraus erhaltene Ergebnis für die Dickenänderung bei der (i+1)-ten Berechnung angewendet wird. Dies kann man so lange durchführen, bis ein Abbruchkriterium erfüllt wird, beispielsweise wenn die Differenz des Ergebnisses für die Dickenänderung bei zwei aufeinanderfolgenden Iterationsschritten unter einer entsprechend festgelegten Grenze liegt. Diese Grenze kann beispielsweise über eine konstante Differenz festgelegt werden, die einer für die Behandlung angemessene Genauigkeit der Refraktionskorrektur entspricht.

Vernachlässigt man die Dickenänderung der Augenhornhaut, was für ein vereinfachtes Verfahren durchaus zulässig ist, kann F in Gleichung (2) für eine vereinfachte Berechnung auch gleich Null gesetzt, also vernachlässigt und weggelassen werden. Man erhält überraschenderweise folgende einfache Gleichung für die Brechkraft der modifizierten Hornhaut 5*:

$$B_{CV}{}^* = B_{CV} + B_{BR} / (1 - B_{BR} \cdot d_{HS})$$

Aus dieser Gleichung ergibt sich für den Fachmann auf einfache Art und Weise mittels der Gleichung $B_{CV}{}^* = (n-1) / R_{CV}{}^*$ der Radius $R_{CV}{}^*$ der Hornhautvorderfläche 15*, der nach der Modifikation vorliegen muß, um die gewünschte Fehlsichtigkeitskorrektur zu erhalten, zu: $R_{CV}{}^* = 1 / ((1/R_{CV}) + B_{BR} / ((n_c-1) (1 - d_{HS} \cdot B_{BR})))$

Für das Volumen, dessen Entfernung die obige Krümmungsänderung der Hornhautvorderfläche 15 bewirkt, wird nun die das Volumen isolierende Grenzfläche festgelegt. Dabei ist vorzugsweise zu berücksichtigen, daß sich der Durchmesser des zu korrigierenden Bereichs und damit der Durchmesser des zu entnehmenden Volumens möglichst über die Pupillengröße bei dunkelangepaßtem Auge erstrecken sollte.

In einer ersten Variante wird mittels dem Fachmann bekannter numerischer Methoden eine Freifläche definiert werden, die ein Volumen umschreibt, dessen Entfernung die Krümmungsänderung bewirkt. Dazu wird entlang der z-Achse die Dickenänderung ermittelt, die zur gewünschten Krümmungsmodifikation nötig ist. Daraus ergibt sich das Volumen als Funktion von r, $\varphi$ (in Zylinderkoordinaten) und daraus wiederum dessen Grenzfläche.

Eine einfache analytische Rechnung liefert die folgende zweite Variante, bei der die Grenzfläche des Volumens durch zwei Teilflächen aufgebaut wird, eine zur Hornhautoberfläche 15 hinliegende anteriore Teilfläche und eine gegenüberliegende posteriore Teilfläche. Die entsprechenden Verhältnisse zeigt Figur 5. Das Volumen 18 ist zur Hornhautvorderfläche 15 hin durch eine anteriore Schnittfläche 19 begrenzt, die in konstantem Abstand $d_F$ unter der Hornhautvorderfläche 15 liegt. Diese anteriore Schnittfläche 19 wird in Analogie zur Laserkeratomen auch als Flap-Fläche 19 bezeichnet, da sie dort dazu dient, in Kombination mit einem Öffnungsschnitt zum Rand hin die Augenhornhaut 5 eine Lamelle in Form eines "Flap" von der darunterliegenden Hornhaut 5 abheben zu können. Diese Art der Entnahme des zuvor isolierten Volumens 18 ist natürlich auch hier möglich.

Die anteriore Schnittfläche 19 hat einen Krümmungsverlauf, der um $d_F$ unter der Hornhautvorderfläche 15 liegt. Ist diese sphärisch, ist kann für die Flap-Fläche 19 ein Krümmungsradius angegeben werden, der um $d_F$ geringer ist als der Krümmungsradius $R_{CV}$. Wie später für bevorzugte Varianten beschrieben wird, kann bei der Erzeugung der Schnittfläche 19 durch ein Kontaktglas dafür gesorgt werden, daß die Hornhautvorderfläche 15 zum Zeitpunkt der Schnittflächenerzeugung sphärisch ist, so daß das Muster der Zielpunkte eine sphärische Schnittfläche bewirkt. Die Relaxation des Auges 3 nach Abnahme des Kontaktglases mag dann zwar zu einer nicht-sphärischen Schnittfläche 19 führen, sie hat aber dennoch konstanten Abstand zur Hornhautvorderfläche 15 bzw. 15*. Dies wird später noch erläutert.

Posterior ist das Volumen 18, das aus der Hornhaut 5 entfernt werden soll, durch eine posteriore Schnittfläche 20 begrenzt, die schon grundsätzlich nicht zur Hornhautvorderfläche 15 in konstantem Abstand sein kann. Die posteriore Schnittfläche 20 wird deshalb so ausgebildet sein, daß das Volumen 18 in Form eines Lentikels vorliegt, weshalb die posteriore Schnittfläche 20 auch als Lentikel-Fläche 20 bezeichnet wird. In Figur 5 ist sie exemplarisch als ebenfalls sphärische Fläche mit einem Krümmungsradius $R_L$ eingezeichnet, wobei natürlich das Zentrum dieser Krümmung nicht mit dem Krümmungszentrum der in Figur 5 ebenfalls sphärischen Hornhautvorderfläche 15 zusammenfällt.

Figur 6 zeigt die Verhältnisse nach Entfernung des Volumens 18. Der Radius der modifizierten Hornhautvorderfläche 15* beträgt nun $R_{CV}$* und kann beispielsweise gemäß den zuvor beschriebenen Gleichungen berechnet werden. Die Dicke $d_L$ des entnommenen Volumens 18 ist dabei maßgeblich für die Radiusänderung, wie Figur 7 verdeutlicht. In dieser Figur sind als weitere Größen noch die Höhe $h_F$ der durch die anteriore Schnittfläche 19 definierten Kugelkappe, die Höhe $h_L$ der durch die posteriore Schnittfläche 20 definierten Kugelkappe sowie die Dicke $d_L$ des zu entfernenden Volumens 18 eingezeichnet.

Die posteriore Schnittfläche 20 legt aufgrund des konstanten Abstandes zwischen Hornhautvorderfläche 15 und anteriorer Schnittfläche 19 den Krümmungsverlauf der Hornhautvorderfläche 15* nach Entfernung des Volumens 18 fest. Somit wird die posteriore Schnittfläche 20 z.B. bei einer zylindrische Parameter berücksichtigenden Fehlsichtigkeitskorrektur einen winkelabhängigen Krümmungsradius haben. Für die in Figur 7 gezeigte Lentikel-Fläche 20 gilt allgemein:

$$R_L(\varphi) = R_{CV}^{\cdot}(\varphi) - d_F \, ,$$

bzw. in Zylinderkoordinaten $(z, r, \varphi)$

$$z_L(r, \varphi) = R_L(\varphi) - (R_L^2(\varphi) - r^2)^{1/2} + d_L + d_F.$$

Ohne Berücksichtigung eines Astigmatismus entfällt die Abhängigkeit von $\varphi$ und die Lentikel-Fläche 20 ist sphärisch. Die Lentikel-Fläche 20 besitzt aber, geht man vom Bedarf für eine zylindrische Fehlsichtigkeitskorrektur aus, in der Regel auf verschiedenen Achsen unterschiedliche Krümmungsradien, wobei diese natürlich meist den gleichen Scheitelpunkt haben.

Damit wird weiter automatisch deutlich, daß im Fall einer Zylinderkorrektur die theoretische Schnittlinie zwischen Flap-Fläche 19 und Lentikel-Fläche 20 nicht in einer Ebene, d.h. bei konstanten z-Koordinaten liegt. Der kleinste Krümmungsradius der Lentikel-Fläche 20 liegt bei $\varphi = \theta + \pi n/2$, der größte natürlich auf der Achse $\theta$ der zylindrischen Fehlsichtigkeit, d.h. bei $\varphi = \theta$. Bei einer Übersichtigkeitskorrektur fallen anders bei der Darstellung der Figur 7 der Scheitelpunkt von Flap-Fläche 19 und Lentikel-Fläche 20 zusammen und die Lentikel-Fläche 20 ist stärker gekrümmt, als die Flap-Fläche 19. Die Dicke $d_L$ des Lentikels ergibt sich als Randdicke.

Das als Lentikel aufzufassende Volumen 18 hat bei $\varphi = \theta = \pi/2$ die geringste Randdicke, da sich dort Lentikel-Fläche 20 und Flap-Fläche 19 schneiden. Bei allen anderen Werten für $\varphi$ ist eine endliche Randdicke gegeben, wenn eine gegebene z-Koordinate als untere Grenze der Lentikel-Fläche 20 angesetzt wird.

Alternativ kann neben der Flap-Fläche 20 und der Lentikel-Fläche 19 zusätzliche Randfläche vorgesehen werden, welche das Volumen 18 im Schnittbereich von Flap-Fläche 20 und der Lentikel-Fläche 19 umrandet bzw diese Flächen dort verbindet, wo sie bei einer gegebenen z-Koordinate nicht zusammenlaufen. Der Schnitt dieser Randfläche wird ebenfalls mit dem gepulsten Laserstrahl ausgefürt. Die Randfläche kann beispielsweise eine zylindrische Form haben, die jedoch auch eine elliptische Form (in der Aufsicht) oder auch eine konische Form (in der Seitenansicht haben kann).

Die in den Figuren gezeigte Ausbildung des Volumens 18 als durch eine anteriore Schnittfläche 19 mit konstantem Abstand zur Hornhautvorderfläche 15 sowie eine posteriore Schnittfläche 20 begrenzt, ist nur eine Variante zur Begrenzung des Volumens 18. Sie hat jedoch den Vorteil, daß die optische Korrektur wesentlich nur durch eine Fläche (die Lentikelfläche 20) festgelegt wird, so daß die analytische Beschreibung der anderen Teilfläche der Grenzfläche einfach istr.

Weiter sind optimale Sicherheitsmargen hinsichtlich des Abstandes des Volumens zur Hornhautvorderfläche 15 und Hornhautrückfläche 16 bietet. Die Restdicke $d_F$ zwischen anteriorer Schnittfläche 19 und Hornhautvorderfläche 15 kann konstant auf einen Wert von beispielsweise 50 bis 200 $\mu$m eingestellt werden. Insbesondere kann sie so gewählt sein, daß das schmerzempfindliche Epithel in der Lamelle verbleibt, die durch die Flap-Fläche 19 unter der Hornhautvorderfläche 15 gebildet ist. Auch steht die Ausbildung der sphärischen Flap-Fläche 19 in Kontinuität mit bisherigen Keratometerschnitten, was für die Akzeptanz der Methode vorteilhaft ist.

Nach Erzeugen der Schnittflächen 19 und 20 wird dann das derart isolierte Volumen 18 aus der Hornhaut 5 entfernt. Dies ist schematisch in Figur 8 dargestellt, die zudem verdeutlicht, daß die Schnittflächen 19 und 20 durch Einwirkung des in einem Fokuskegel 21 einfallenden Behandlungslaserstrahls erzeugt werden, beispielsweise durch Aneinanderreihung von Plasmablasen, so daß in einer bevorzugten Ausführungsform die Flap-Schnittfläche 19 und die Lentikel-Schnittfläche 20 durch geeignete dreidimensionale Verstellung der Fokuslage der gepulsten Laserstrahlung 2 erzeugt werden.

Alternativ kann in einer vereinfachten Ausführungsform aber auch lediglich die Flap-Fläche 19 durch Zielpunkte, die die gekrümmte Schnittfläche 19 in konstantem Abstand zu Hornhautvorderfläche 15 definieren mittels gepulster Laserstrahlung gebildet werden und die Entfernung des Volumens 18 erfolgt durch Laserablation, beispielsweise durch Verwendung eines Excimers-Laserstrahls. Hierzu kann die Lentikel-Fläche 20 als Grenzfläche des Abtrages definiert werden, auch wenn das nicht zwingend erforderlich ist. Das Behandlungsgerät 1 arbeitet da wie ein bekanntes Laserkeratom, allerdings wird die Schnittfläche 19 an gekrümmter Hornhaut erzeugt. Die vorangehend bzw. nachfolgend beschriebenen Merkmale sind auch in solchen Varianten möglich, insbesondere was die Bestimmung der Begrenzungsfläche, deren geometrische Definition und die Ermittlung von Steuerparametern angeht.

Erzeugt man sowohl die Lentikel-Fläche 20 als auch die Flap-Fläche 19 mittels gepulster Laserstrahlung, ist es zweckmäßig, die Lentikel-Fläche 20 vor der Flap-Fläche 19 auszubilden, da das optische Ergebnis bei der Lentikel-Fläche 20 besser (wenn nicht überhaupt erst zu erreichen) ist, wenn oberhalb der Lentikel-Fläche 20 noch keine Veränderung der Hornhaut 5 eintrat.

Das Entfernen des durch die gepulste Laserstrahlung isolierten Volumens 18 kann, wie in Figur 8 angedeutet, durch einen Randschnitt 22 erreicht werden, der es erlaubt, das Volumen 18 in Richtung eines in Figur 8 eingezeichneten Pfeiles 23 herauszuziehen. Alternativ kann der Randschnitt 22 aber so ausgebildet werden, daß er die anteriore Schnittfläche 19, d. h. die Flap-Fläche 19, in Form eines Ringes mit der Hornhautvorderfläche 15 verbindet, wobei der Randschnitt allerdings nicht vollständig um einen Winkel von 360° umläuft. Die derart isolierte Lamelle bleibt in einem schmale Bereich mit dem übrigen Gewebe der Hornhaut 5 in Verbindung. Diese Verbindungsbrücke dient dann als Gelenk, um die ansonsten isolierte Lamelle von der Hornhaut 5 abzuklappen und das dadurch zugängige, bereits isolierte Volumen 18 vom Rest der Augenhornhaut 5 abnehmen zu können. Die Lage der Verbindungsbrücke ist bei Erzeugung der Steuerdaten bzw. der Zielpunkte vorgebbar. Das beschriebene Vorgehen bzw. Gerät realisiert also unter diesem Gesichtspunkt die Isolierung des Volumens 19 innerhalb der Hornhaut 5 und das Erzeugen einer mit der restlichen Augenhornhaut über eine Gewebebrücke verbundenen Lamelle als Deckel über dem Volumen. Der Deckel kann abgeklappt und das Volumen 18 entnommen werden.

Für die Erzeugung der Schnittflächen 19 und 20 können die Zielpunkte nun auf verschiedenste Art und Weise angeordnet werden. Im Stand der Technik ist beispielsweise in der WO 2005/011546 zur Erzeugung von Schnittflächen in der Augenhornhaut beschrieben, daß spezielle Spiralen eingesetzt werden können, die beispielsweise um eine im wesentlichen senkrecht zur optischen Achse (z-Achse) liegende Hauptachse in Art einer Schraubenlinie verlaufen. Auch ist die Verwendung eines Scanmusters bekannt, das die Zielpunkte zeilenweise anordnet (vgl. WO 2005/011545). Diese Möglichkeiten können selbstverständlich zur Erzeugung der oben definierten Schnittflächen verwendet und mit den nachfolgend erläuterten Transformationen werden.

Die Verstellung der Lage des Fokus in der Augenhornhaut erfolgt mittels der in Figur 3 schematisch dargestellten dreidimensionalen Ablenkeinrichtung, die zur Verstellung des Fokus in z-Richtung die Verschiebung von Linsen oder anderer optisch wirksamer Elemente einsetzt. Nun ist die Verstellung von Linsen o.ä. in der Regel nicht so schnell möglich, wie die Verschwenkung von Spiegeln, wie sie in der Regel im xy-Scanner Einsatz finden. Meist ist deshalb die Verstellgeschwindigkeit des z-Scanners begrenzend für die Geschwindigkeit, mit der die Schnittflächen in der Augenhornhaut erzeugt werden können. Zur möglichst schnellen Erzeugung der Schnittflächen 18 und 19 wird deshalb in einer bevorzugten Ausführungsform der Fokus jeweils entlang einer spiralförmigen Bahn geführt, wobei je eine Spirale in der räumlich gekrümmten Schnittfläche liegt. Während des Schreibens der Spirale wird also der z-Scanner so verstellt, daß die Arme der Spirale der räumlich gekrümmten Schnittfläche folgen.

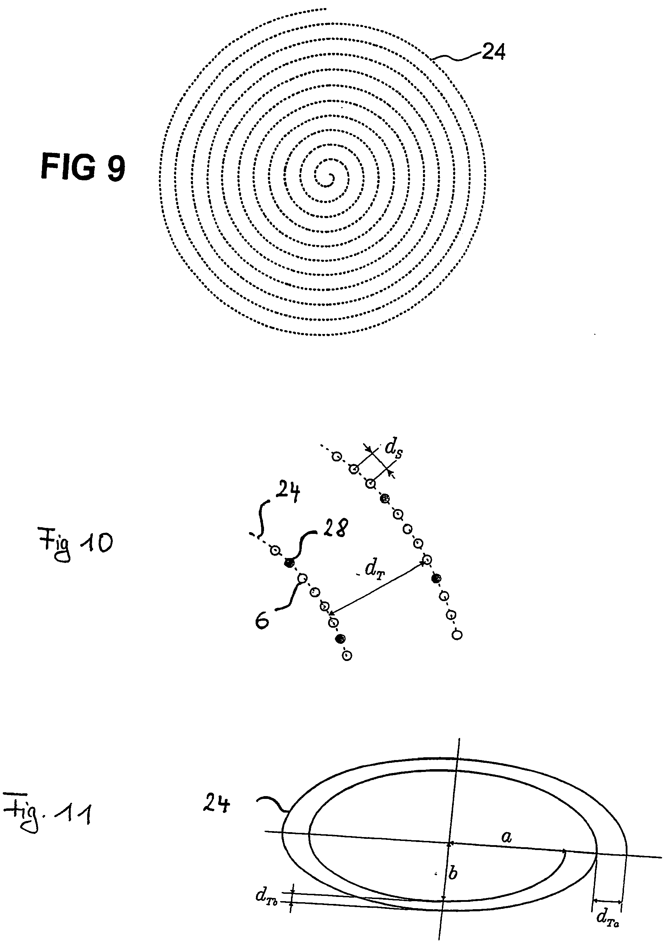

Figur 9 zeigt exemplarisch eine Bahnkurve 24 als Spirale, die in der gezeigten Darstellung als Kreisspirale ausgebildet ist. Der Radius der dargestellten ebenen Spirale nimmt in Kreiskoordinaten mit steigendem Drehwinkel $\varphi$ zu, so daß gilt:

$$(4) \qquad r(\varphi) = \varphi \cdot d_T / (2\pi)$$

In dieser Gleichung bezeichnet $d_T$ den Abstand der Spiralarme; er ist in Figur 10 dargestellt, die einen vergrößerten Ausschnitt der Figur 9 zeigt. Der Abstand der einzelnen Spots 6, auf die gepulste Laserstrahlung fokussiert wird und an denen durch einen Laserpuls beispielsweise eine Plasmablase erzeugt wird, ist in der Spirale konstant gleich $d_S$, so daß für den Winkelabstand $\Delta\varphi$ der einzelnen Spots 6, an denen ein Laserpuls in das Gewebe eingebracht wird, gilt:

$$\Delta\varphi = d_S / r$$

Da, wie bereits erwähnt, die Lentikel-Fläche 20 in der Regel nichtsphärisch ist, ist die Bahnkurve 24, entlang der der Laserfokus verstellt wird, eine elliptische Spirale, für die natürlich kein konstanter Abstand der Spiralarme mehr gegeben ist. Entlang der Hauptachsen a und b kann aber ein jeweiliger Bahnabstand $d_{Tb}$ sowie $d_{Ta}$ definiert werden, wie Figur 11 zeigt.

In Figur 10 sind die Spots 6 dargestellt, um die Lage des Fokus für die einzelnen Laserpulse erkennen zu lassen. Tatsächlich weiten sich die Plasmablasen natürlich nach Einbringung des jeweiligen Laserpulses so weit auf, daß die Schnittfläche erzeugt wird und die Bahnkurve 24 ist in der Schnittfläche dann nicht mehr zu erkennen.

Zur Vorbereitung des chirurgischen Verfahrens muß nach der Definition der Schnittflächen 19 und 20 nun die Definition der Bahnkurven 24 erfolgen, mit denen die Schnittflächen erzeugt werden.

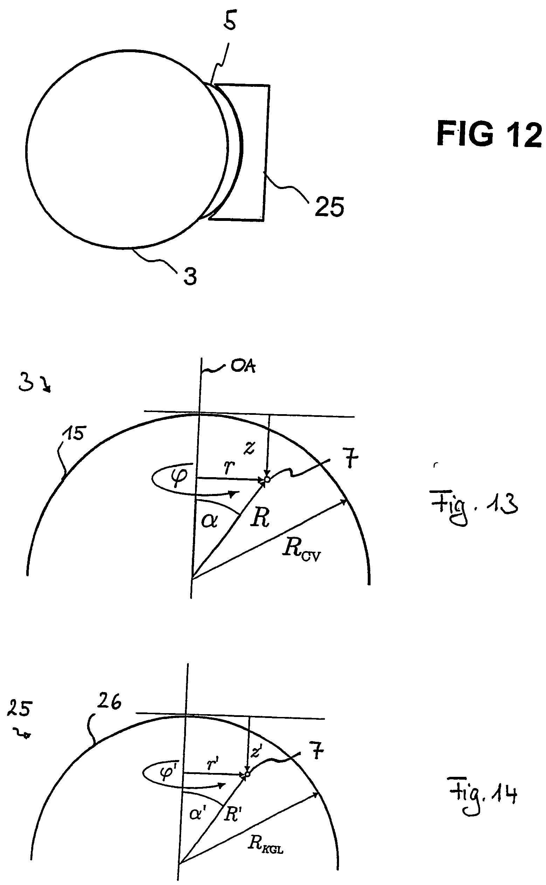

Bei der Bestimmung der Bahnkurven 24 ist natürlich zu berücksichtigen, daß letztendlich das Volumen 18 im Auge im Normalzustand definiert sein soll. Die Schnittflächen 19 und 20, wie sie bislang erläutert wurden, betreffen das natürliche Auge. Es ist nun aber zu berücksichtigen, daß das Behandlungsgerät 1 aus Gründen der Fixierung des Auges mit einem Kontaktglas 25 arbeitet, das wie in Figur 12 gezeigt ist, auf die Hornhautvorderfläche 15 der Augenhornhaut 5 aufgesetzt wird. Das Kontaktglas 25, das bereits Gegenstand mehrerer Patentpublikationen ist (exemplarisch sei beispielsweise auf die WO 2005/048895 A verwiesen), ist für die hier vorliegende Beschreibung des Behandlungsgerätes 1 bzw. der damit in Zusammenhang stehenden Verfahren zur Vorbereitung und/oder Durchführung des chirurgischen Eingriffes allerdings nur insoweit von Interesse, als es der Hornhautvorderfläche 15 zum einen eine definierte Krümmung verleiht und zum anderen die Augenhornhaut 5 gegenüber dem Behandlungsgerät 1 räumlich in einer vordefinierten Lage hält. Hinsichtlich der sphärischen Krümmung der Kontaktfläche des Kontaktglases 25 unterscheidet sich der hier beschriebene Ansatz jedoch deutlich von dem Ansatz, wie er beispielsweise in der WO 2003/002008 A beschrieben ist, der ein planes Kontaktglas verwendet, welches die Augenhornhaut flachdrückt.

Wird das Auge an das Kontaktglas 25 mit sphärischer Kontaktfläche angepreßt, kommt es zu einer räumlichen Deformation des Auges. Da die Kornea regelmäßig nur tangential kompressibel ist, also bei einem solchen Anpressen ihre Dicke nicht ändert, entspricht das Anpressen einer Transformation vom Koordinatensystem des Auges, wie es in Figur 13 dargestellt ist, in das Koordinatensystem des Kontaktglases, das in Figur 14 gezeigt ist. Dieser Zusammenhang ist dem Fachmann aus der WO 2005/011547 A1 bekannt, deren Offenbarungsgehalt diesbezüglich vollumfänglich eingebunden sein soll. In den Figuren 13 und 14 bezeichnen mit einem Apostroph versehene Koordinaten die Koordinaten des auf das Kontaktglas 25 bzw. dessen dem Auge zugewandte Kontaktglasunterseite 26 bezogenen Größen.

Das Kontaktglas hat aber noch einen weiteren Vorteil. Durch das Anpressen an die sphärische Kontaktglasunterseite 26 ist automatisch auch die Hornhautvorderfläche 15 sphärisch. Die in konstantem Abstand unter der Hornhautvorderfläche 15 liegende anteriore Schnittfläche 19 ist damit bei angepreßtem Kontaktglas ebenfalls sphärisch, was zu erheblich vereinfachter Ansteuerung führt. Es ist deshalb völlig unabhängig von anderen Merkmalen bevorzugt, ein Kontaktglas 25 mit sphärischer Kontaktglasunterseite 26 zu verwenden und das Volumen durch eine anteriore Schnittfläche 19 sowie eine posteriore Schnittfläche zu begrenzen, wobei für die anteriore Schnittfläche Zielpunkte vorgegeben sind/werden, die diese Schnittfläche als sphärische Fläche in konstantem Abstand $d_F$ unter der Hornhautvorderfläche 15 ausbilden. Für die posteriore Schnittfläche sind/werden Zielpunkte vorgegeben, die einen Krümmungsverlauf definieren, welcher bei relaxiertem Auge, also nach Abnehmen des Kontaktglases, bis auf den Abstand $d_F$ zur Hornhautvorderfläche dem zur Fehlsichtigkeitskorrektur gewünschten entsprechen. Analoges gilt für das Verfahren zur Definition der Zielpunkte bzw. das Operationsverfahren.

Die Darstellungen in den Figuren 13 und 14 zeigen die Koordinatentransformation, die am Auge durch das Aufsetzten bzw. Abnehmen des Kontaktglases auftritt. Sie enthalten sowohl Kugelkoordinaten (R, $\alpha$, $\varphi$) bezogen auf den Ursprung der gekrümmten Fläche (Hornhautvorderfläche 15 bzw. Kontaktglasunterseite 26) als auch Zylinderkoordinaten (r, z, $\varphi$) bezogen auf den durch den Durchtrittspunkt der optischen Achse OA definierten Scheitelpunkt der Hornhautvorderfläche 15 bzw. der Kontaktglasunterseite 26.

Bei der Koordinatentransformation vom auf das Auge bezogenen Koordinatensystem, wie es in Figur 13 dargestellt ist, in das auf das Kontaktglas bezogene System gemäß Figur 14 bleiben die Bogenlänge, d.h. $\alpha \cdot R$, die radiale Tiefe ($R_{CV}$ - R) sowie der Winkel $\varphi$ erhalten. Die Transformation der für das natürliche Auge, d.h. im Koordinatensystem der Figur 13, zugrundegelegten Formen der Schnittflächen 19 und 20 ist somit ein wichtiger Schritt bei der Berechnung der Ansteuergrößen für die dreidimensionale Fokusverstelleinrichtung. Sie verläuft grundsätzlich anders als bei einem ebenen Kontaktglas, in dem z.B. die Flap-Fläche 19 zu einer Ebene entartet. Im Wesentlichen ist nur die Form für die Schnittfläche 20 zu transformieren, da die Schnittfläche 19 lediglich in konstantem Abstand $d_F$ zur Hornhautvorderfläche 15 auszubilden ist. Die Schnittfläche 19 ist also im transformierten System eine Sphäre mit einem gegenüber der Kontaktglasunterseite um $d_F$ reduzierten Krümmungsradius $R_F$.

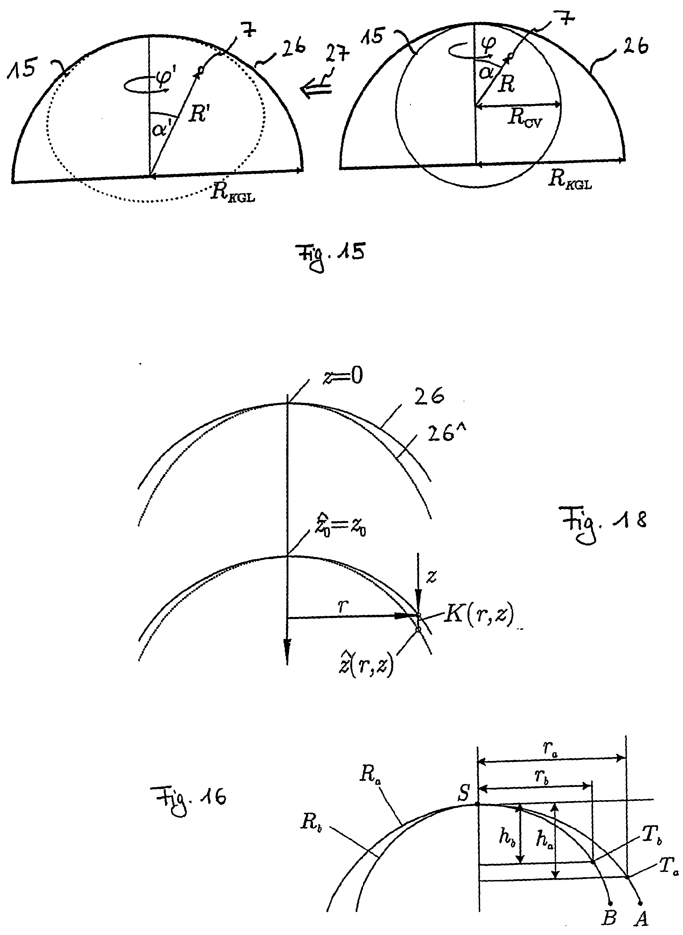

Das Anpressen der Hornhaut 5 des Auges 3 an die sphärisch gekrümmte Kontaktglasunterseite 26 ist in Figur 15 veranschaulicht. Dort zeigt die rechte Darstellung schematisch den Zustand, wenn die Kontaktglasunterseite 26 nur am Scheitelpunkt in Kontakt mit der Hornhautvorderfläche 15 ist. Zur Verdeutlichung der geometrischen Beziehungen ist die Hornhautvorderfläche 15 schematisch in Figur 15 als Kreis eingezeichnet, obschon natürlich die sphärische Krümmung nur in einem kleineren Kreisabschnitt vorliegt. Das Anpressen des Kontaktglases 25 auf die Hornhaut 5 bewirkt den durch Pfeil 27 symbolisierten Übergang zum Zustand der linken Seite der Figur 15. Das Abnehmen des Kontaktglases 25 bewirkt eine Relaxation des Auges 3 entgegen der Richtung des Pfeiles 27

Aufgrund der geschilderten Rahmenbedingungen transformieren sich für jeden Punkt in der Augenhornhaut 5 die Koordinaten von dem in Figur 13 dargestellten System in das System der Figur 14. Dies wird nun bei der Wahl der Ansteuerwerte für die Fokusverstellung dahingehend zugrundegelegt, daß die Schnittflächen 19 und 20 im transformierten Kontaktglassystem zu beschreiben sind, da sie nur dann nach Abnehmen des Kontaktglases 26, d.h. nach RückTransformation in das natürliche Koordinatensystem des Auges, die gewünschten Formen haben. Da das Anlegen der Hornhautvorderfläche 15 in der Regel durch Ansaugen mittels Unterdruck bewirkt wird, wird die geschilderte Transformation nachfolgend auch als Ansaugtransformation bezeichnet.

Zum Schneiden der Flap-Fläche 19, die wie erwähnt sphärisch ist, wird nun folgende Geschwindigkeit der Verstellung des z-Scanners, d.h. folgende Vorschubgeschwindigkeit in z-Richtung eingestellt:

$$(5) \qquad v_Z(t) = d_S \bullet f_L \bullet d_T / (2\pi \bullet (R_F^2 - t \bullet d_S \bullet f_L \bullet d_T / \pi))^{\frac{1}{2}},$$

wobei $f_L$ die Frequenz der Laserpulse der Laserstrahlung 2 ist. Gleichung (5) setzt voraus, daß die z-Geschwindigkeit $v_Z$ frei eingestellt und kontinuierlich verändert werden kann.

Möchte man eine Sphäre mit einer Geschwindigkeit $v_Z$ schreiben, die aus einer Gruppe diskreter Geschwindigkeiten gewählt ist, was in der Regel dann der Fall ist, wenn der z-Scanner mittels eines Schrittmotors angetrieben ist, erhält man als Zeitabhängigkeit der Radialfunktion r(t):

$$(6) \qquad r(t) = [d_S \bullet f_L \bullet d_T \bullet t/\pi - (d_S \bullet f_L \bullet d_T \bullet t)^2/(2\pi \bullet R_F)^2]^{1/2}$$

sowie für die Winkelfunktion

$$(7) \qquad \varphi(t) = [4\pi \bullet d_S \bullet f_L \bullet t/d_T - (d_S \bullet f_L \bullet t)^2/R^2]^{1/2}.$$

Die $t^2$-Terme unter der Wurzel der Radial- wie der Winkelfunktion zeigen, daß keine ideale archimedrische Spirale mehr geschrieben wird, die Bahn- und Spotblasen-Abstände variieren also zugunsten der nur in Stufen veränderlichen z-Geschwindigkeit.

Wünscht man bei der Fokusverstellung einen konstanten z-Vorschub, ergibt sich nicht, wie mit der Geschwindigkeit gemäß Gleichung (4) eine Sphäre, sondern ein Paraboloid, und es gilt:

$$(8) \qquad z(r) = [v_Z/(d_S \bullet d_r)][r^2 \bullet \pi/f_L]$$

Erwähnterweise kann in manchen Behandlungsgeräten 1 die Geschwindigkeit, mit der der z-Scanner den Fokus in z-Richtung verschiebt, nur innerhalb eines Satzes diskreter Geschwindigkeiten verstellt werden. Möchte man dann eine bestimmte Parabel mit einer gegebenen Geschwindigkeit $v_Z$ beschreiben, muß das Produkt $d_S \bullet d_T$ entsprechend gewählt werden, so daß die erste eckige Klammer der Gleichung (8) den gewünschten Wert einnimmt. Der Abstand der Bahnen, definiert durch $d_T$, sowie der Spotabstand entlang der Bahnbeschrieben durch $d_S$, sind also geeignet zu variieren, um eine bestimmte Parabel mit gegebenen $v_Z$ zu schreiben.

Jede der Gleichungen (5), (6)/(7) und (8) kann bei der Ermittlung der Zielpunkte und damit de Ansteuerung der Fokusverstellung verwendet werden, wobei dann natürlich die entsprechende Spiralform/Flächenform zugrundezulegen ist. Wenn nachfolgend davon gesprochen wird, daß die Gleichungen bei der Ansteuerung verwendet werden, ist darunter insbesondere zu verstehen, daß mittels der Gleichungen die Zielpunkte ermittelt werden, die kann z.B. durch Auswerten der Funktionsgleichungen zu äquidistanten Zeitpunkten geschehen. Die Geschwindigkeitsgleichungen werden in einer Variante dazu verwendet, sicherzustellen, daß die ermittelten Zielpunkte keine Verstellgeschwindigkeiten bedingen, die von der Fokusverstelleinrichtung gar nicht realisierbar sind.

Für die eingangs erwähnten und wie beschrieben ermittelten Formen der Flächen 19 und 20 wird nun eine Spirale in die jeweilige Fläche gelegt. Sie Spirale wird durch eine Ansteuerung der beschriebenen Art geschrieben. Die Berechnung der z-Geschwindigkeit sowie der r- und $\varphi$-Geschwindigkeit berücksichtigt dabei, welche Flächenform die Fläche 19 bzw. 20 hat.

Die Lentikel-Fläche 20 ist sphärisch, wenn keine Zylinderkorrektur vorgenommen werden soll. Man verwendet deshalb die Ansteuerung gemäß Gleichungen (4)/(5) oder (6)/(7) um diese Sphäre zu erzeugen. Allerdings kann eine Sphäre bekanntermaßen auch durch ein Paraboloid approximiert werden. Es ist deshalb in einer Variante vorgesehen, die eine Sphäre auf dem Fachmann bekannter Weise durch ein Paraboloid anzunähern und die Ansteuerung gemäß Gleichung (8) vorzunehmen.

Durch die Ansaugtransformation gemäß Figur 15 verändert sich die Geometrie der Lentikel-Fläche 20. Die Lentikel-Fläche 20 muß im Koordinatensystem des Kontaktglases 25 den Krümmungsverlauf der korrigierten Hornhautvorderfläche 15* aufweisen. Sie kann nicht auf einen Krümmungsmittelpunkt bezogen werden, der mit dem Krümmungsmittelpunkt des Kontaktglases zusammenfällt. Die gemäß Gleichung (2) definierte Krümmung wird also bezüglich der Ansaugtransformation umgerechnet.

Der in Gleichung (2) definierte Krümmungsradius ist natürlich eine Funktion von $\phi$. Wie bereits erwähnt und in der Augenoptik üblich, können zwei Krümmungsradien $r_a$ bzw. $r_b$ angegeben werden: einer auf der Achse $\theta$ der zylindrischen Fehlsichtigkeit und einer für eine Achse rechtwinklig dazu. Rechentechnisch ist es besonders günstig, die sich somit im allgemeinen Fall einstellende toroidale Krümmung durch eine Parabel zu approximieren, so daß die Lentikel-Fläche 20 durch ein Paraboloid angenähert wird. Dies geschieht vorzugsweise vor der Anpreßtransformation kann aber auch danach durchgeführt werden.

Die Annäherung erfolgt dadurch, daß man für die zwei Krümmungsradien jeweils eine Parabel sucht, die sowohl durch den Scheitelpunkt der Lentikel-Fläche 20 als auch durch einen möglichst am Rand gelegenen Punkt läuft. Die entsprechenden Verhältnisse im Koordinatensystem des Auges zeigt Figur 16. Diese Figur zeigt die sphärische Lentikel-Fläche 20 vor der Anpreßtransformation. In der Figur sind die Schnitte durch die im verallgemeinerten Fall toroidale Lentikel-Fläche 20 entlang der zwei Halbachsen a und b übereinandergelegt. Die entsprechenden Kurven sind mit A bzw. B bezeichnet und sind kreisförmig mit einem Krümmungsradius $r_a$ bzw. $r_b$. Auf jeder Kurve ist ein Randpunkt T in Zylinderkoordinaten durch den entsprechenden Radius r sowie die Höhe h beschrieben, wobei diese Parameter auf den Scheitelpunkt S bezogen sind, der für die zwei Halbachsenschnitte identisch ist. Der Punkt $T_a$ ist also durch den Radius $r_a$ sowie die Höhe $h_a$ gekennzeichnet. Analoges gilt für $T_b$.

Es wird nun eine Parabel gesucht, für die gilt $h = k \cdot r^2$. Die dadurch erhaltenen Parabelparameter $h_a$ für die Parabel entlang der großen Halbachse a sowie $k_b$ für die Parabel entlang der kleinen Halbachse b definieren das Paraboloid, das dann unter Verstellung des Fokuspunktes in z-Richtung geschrieben wird, beispielsweise mittels eines konstanten z-Vorschubes (vgl. Gleichung (7)) bzw. die mit einer Auswahl der z-Geschwindigkeit aus einem Satz diskreter Geschwindigkeiten gewählt wird (Modifikation zur Gleichung (7)). Die in Figur 16 dargestellten Schnitte der toriodalen Lentikel-Fläche 20 entlang der kleinen Hauptachse b sowie der großen Hauptachse a beziehen sich auf die Darstellung im Koordinatensystem des Auges.

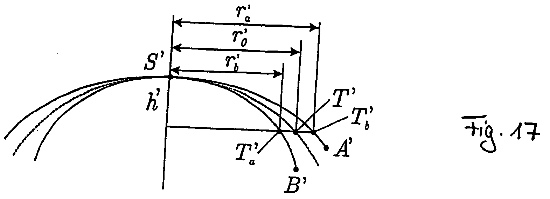

Wenn die Approximation durch Parabelgleichungen nach der Anpreßtransformation durchgeführt wurde, treten dort natürlich die transformierten Werte auf. Man kann die explizire Berechnung von transformierten Werten an dieser Stelle vermeiden, wenn die Approximation zuerst erfolgt und die dabei gefundenen Parabelparameter der Anpreßtransformation in das Koordinatensystem des Kontaktglases unterworfen werden, wonach dann die in Figur 17 dargestellten Verhältnisse vorliegen.

Die Spezifikation der Parabelparameter lautet im Koordinatensystem des Auges gemäß Figur 16 wie folgt:

$$(9) \qquad k_a = (\, z(T_a) - z(S) \,) / r(T_a)^2,$$

$$(10) \qquad k_b = (\, z(T_b) - z(S) \,) / r(T_b)^2.$$

In den Gleichungen (9) und (10) bezeichnet z(S) die z-Koordinate des Punktes S. Legt man den Koordinatensystemursprung, wie in den bisherigen Figuren, in den Scheitelpunkt, ist die z-Koordinate Null. Die Koordinate $z(T_a)$ bzw. $z(T_b)$ sowie $r(T_a)$ bzw. $r(T_b)$ sind die z- bzw. r-Koordinaten des entsprechenden Punktes $T_a$ bzw. $T_b$ im zylindrischen Koordinatensystem.

Werden die Parabelparameter $k_a$ bzw. $k_b$ nicht im Koordinatensystem des Auges gemäß Figur 16, sondern im Koordinatensystem des Kontaktglases gemäß Figur 17 benötigt, treten anstelle der Punkte S, $T_a$ und $T_a$ dann die in Figur 17 eingezeichneten transformierten Punkte S', $T_a$', $T_b$'.

Um die Lentikel-Fläche 20 nun im angepreßten Auge 3 durch eine (dann in der Regel elliptische) Spirale mit den Hauptachsen $r_a$' und $r_b$' darzustellen, wird die Spirale aus einer Kreisspirale mit Radius $r_0$' durch Streckung in Richtung $\varphi = \theta$ und Stauchung in Richtung $\varphi = \theta + \pi/2$ konstruiert. Durch die gleichzeitige Stauchung und Streckung bleibt der mittlere Bahn- bzw.

Spotabstand erhalten. Würde man nur in eine Richtung stauchen oder strecken, würde der mittlere Abstand verändert.

Die eigentlichen Radien lassen sich aus dem Radius $r_0$ der Kreisspirale, der in Figur 17 gestrichelt eingezeichnet ist, mit Hilfe der Elliptizität wie folgt berechnet:

$$e' = r_a'/r_b' = (k_8'/k_a')^{1/2}.$$

Die Elliptizität e' ist dabei die Elliptizität der transformierten toriodalen Lentikel-Fläche 20. Die Parameter $k_b'$ sowie $k_a'$ sind durch die Gleichungen (11) und (12), jeweils für die transformierten Punkte S', $T_a'$ und $T_b'$ gegeben. Die Parabelparameter ergeben sich daraus, daß hier die Kreisspirale mit dem Radius $r_0$, aus dem die Lentikel-Fläche 20 konstruiert ist, ein arithmetisches oder geometrisches Mittel der Krümmungen der großen bzw. kleinen Halbachse des Paraboloid sein soll. Wegen $r_0' = (r_a' \cdot r_b')^{1/2}$ erhält man für den Parabelparameter $k = (k_a \cdot k_b)^{1/2}$ sowie die Halbachsen der Ellipse: $r_a' = {}_{r0}' \cdot (e')^{1/2}$ und $r_b' = r_0' \cdot (e')^{-1/2}$.

An dieser Stelle der Ermittlung der Zielpunkte liegen nun zwei Bahnkurven 24 vor, die durch Funktionsgleichungen beschreiben sind. Das Muster der Zielpunkte wird durch Auswertung der Funktionsgleichungen ermittelt.

Allerdings bleibt noch zu berücksichtigen, daß die Fokussierung des Laserstrahls in den Fokus 7 einem Fokuslagenfehler unterliegt. Dieser Fokuslagenfehler ist Eigenschaft des optischen Systems, d. h. beruht auf der verwendeten optischen Realisierung. Er ist im wesentlichen durch das optische Design bestimmt. Aufgrund endlicher Fertigungsgenauigkeiten im Rahmen der erlaubten Toleranz ist der Fokuslagenfehler darüber hinaus geräteindividuell. Er wird deshalb zweckmäßigerweise für jedes Gerät eigenständig bestimmt.

Die Berücksichtigung des Fokuslagenfehlers erfolgt durch eine in der Regel nichtlineare Transformation (nachfolgend auch als NL-Transformation bezeichnet). Es ist deshalb nicht möglich, die NL-Transformation durch Modifikation der Bahnkurvenparameter auszuführen. In einer bevorzugten Ausführungsform wird der Fokuslagenfehler durch eine Korrekturtabelle oder eine Korrekturfunktion ausgedrückt. Sie stammt aus einer Vermessung der Optik des Behandlungsgerätes 1. Die Vermessung kann gerätetypbezogen oder geräteindividuell geschehen. Die Korrekturfunktion kann aus einer Interpolation der Meßresultate z. B. mittels Polynomen oder Splines gewonnen sein. Meist ist der Fokuslagenfehler rotationssymmetrisch bezogen auf die optische Achse. Er hängt dann in Zylinderkoordinaten nur von r und z ab.

Die zuvor mittels der Bahnkurven errechneten Punkte werden in der NL-Transformation so vorverzerrt, daß sie nach der Einbringung der Laserspots mit dem optischen System, das den Fokuslagenfehler aufweist, genau an der gewünschten Stelle liegen. Die Anwendung der vorverzerrten Koordinaten kompensiert also den im optischen System auftretende Fokuslagenfehler.

Die NL-Transformation geht von der Überlegung aus, daß man zu jedem Punkt mit den Koordinaten (z, r) eine um $z_0$ verschobene Kontaktglassphäre findet, auf der dieser Punkt liegt. Der Scheitelpunkt dieser Sphäre ist dann gerade bei $z_0(z, r) = z - z_{KGL}(r)$. Die Wirkung der Vorverzerrung zur Kompensation des Fokuslagenfehlers ist in Figur 18 veranschaulicht. Sie zeigt die Kontaktglasunterseite 26, die im vorliegenden Beispiel sphärisch ist, aber auch eine andere Form haben kann. Aufgrund der Vorverzerrung wird sie zu einer transformierten Kontaktglassphäre 26^. Die die Vorverzerrung des Fokuslagenfehlers berücksichtigenden Parameter sind in Figur 18 durch ein angefügtes Dach "^" symbolisiert. Für einen transformierten Achsenpunkt in der Kornea gilt dann $z_0^{\wedge} = z_0$. Dies trägt der Tatsache Rechnung, daß der Fokuslagenfehler zwar meist rotationssymmetrisch ist, allerdings auch eine Verschiebung in z-Richtung zur Folge hat.

Zur Vorverzerrung werden die berechneten Bahnkurven in individuelle Zielpunkt-Koordinaten für die Spots umgesetzt, welche dann in der in Figur 18 durch K(r, z) symbolisierten Korrekturtransformation, also der NL-Transformation, verschoben werden. Ist der Fokuslagenfehler als Funktion K angeben, muß die Koordinate eines jeden Zielpunktes lediglich mit der Funktion ausgewertet werden, um die Verschiebung bzw. die transformierte Koordinate zu erhalten.

Im Ergebnis liegt dann für die Flächen 19 und 20 jeweils ein Satz Zielpunkt-Koordinaten vor, entlang der der Fokus geführt wird. Durch die NL-Transformation und die Anpreßtransformation liegen die Koordinaten bezogen auf das natürliche, also freie Auge genau in den gewünschten anterioren und posterioren Schnittflächen 19, 20.

Die so erhaltenen Koordinaten für die Zielpunkte müssen noch in Ansteuersignale für die dreidimensionale Ablenkeinheit, z.B. die xy-Scanner sowie den z-Scanner umgesetzt werden. Hierzu wird ein entsprechender funktioneller Zusammenhang oder ein entsprechendes Kennfeld verwendet, daß für die Scanner bekannt ist und gegebenenfalls vorab ermittelt wurde.

Insbesondere für die xy-Scanner, die im Ausführungsbeispiel als Galvanometerspiegel realisiert sind, wurde zuvor die Response-Funktion bestimmt. Ein Beaufschlagen der Galvanometerspiegel mit einem Frequenz-Sweep sowie Messen der tatsächlichen Galvanometerbewegung liefern eine Amplituden- und Phasen-Antwortfunktion. Diese werden bei der Bestimmung der Ansteuersignale berücksichtigt.

Weiter wird zur vereinfachten Ansteuerung nicht für jeden Punkt dem Scanner ein Signal für das anzufahrende Ziel vorgegeben. Statt dessen bewirkt das Steuergerät 12 eine Vorgabe von Stützstellen, die die Bahn des Scanners charakterisieren. Die Punktezahl ist dadurch deutlich reduziert. Dies kann schon nutzbringend bei der NL-Transformation ausgenutzt werden, indem nur für die Bahnkurven nur diejenigen Zielpunkte der Transformation unterworfen werden, die Stützstellen bei der Ansteuerung sein sollen. Die Auswertung der Funktionsgleichungen erfolgt in einer Ausführungsform also mittels eines auf einen zeitlichen Abstands der geringer ist, als der Zeitabstand der Laserpulse.

Es wird somit also vor der NL-Transformation eine Filterung der Bahnkurvenpunkte vorgenommen, die die erwähnten Stützstellen, d.h. Punkte in einer Frequenz der Scanneransteuerung zur Transformation vorsieht. Äquivalent mit einer solchen Filterung ist eine Auswertung der funktionsmäßig beschriebenen Bahnkurven an entsprechend der Scannersteuerung beabstandeten Stützstellen. Hier tritt ein weiterer Vorteil des hier geschilderten funktionsbasierten Ansatzes zu Tage: Die Entscheidung, welche Punkte Zielpunkte bei der Ansteuerung der Fokusverstelleinrichtung sind, muß erst vor der NL-Transformation getroffen werden. Zuvor müssen lediglich die Bahnparameter geeignet umgerechnet werden. Auch liegen erst dann Datensätze mit einer Vielzahl an Punkten vor.

Die Stützstellen definieren somit Zielpunkte, die nur eine Teilmenge der Menge der Punkte bilden, an die ein Laserpuls abgegeben wird. Dies ist in Figur 10 veranschaulicht, in der diejenigen Spots 6, die ein Zielpunkt 28 im Steuerdatensatz sind vorliegt, als schwarze ausgefüllte Kreise eingezeichnet sind.

Dieses Vorgehen hat zudem den Vorteil, daß die Maximalfrequenz $f_S$, die bei der Ansteuerung des Scanners auftritt, sehr viel geringer sind, als die Laserpulsfrequenz fp. Beispielsweise kann mit einer Ansteuerfrequenz von 20 kHz sowie einer Laserpulsfrequenz von 200 kHz gearbeitet werden. Im Ergebnis liegen somit zwischen den Zielpunkten 28, die bei der Ansteuerung des Scanners vorgegeben werden, ein oder mehrere Spots 6, auf die ebenfalls gepulste Laserstrahlung abgegeben wird.

Es erfolgt also nicht nur eine Abgabe von gepulster Laserstrahlung, während sich die Scanner in einem Verstellvorgang befinden, beispielsweise während die Galvanometerspiegel sich bewegen, sondern Laserpulse werden von den Scannern abgelenkt, während diese sich von einem vorgegebenen Zielpunkt zum nächsten bewegen. Um eine möglichst hohe Ablenkgeschwindigkeit zu erreichen, stellt diese Bewegung eine Schwingung dar, die bei perfekten Kreisspiralen (wie sie bei der anterioren Schnittfläche 19 auftreten) sogar eine rein sinusförmige Schwingung ist. Da auch bei der Lentikel-Fläche 20 die tatsächliche Spiralform nur wenig von idealen Kreis- oder elliptischen Spiralen abweicht, können die Scanner nahe ihrer Grenzfrequenz betrieben werden, so daß die beschriebenen Bahnen, entlang derer die Spots angeordnet sind, eine sehr schnelle Schnittflächenerzeugung erlauben.

Nach der Ermittlung der Steuerdatensätze enthaltend der Zielpunkte aus den geschilderten Punktmengen, die für die Bahnkurven erhalten wurden, ist das Vorverfahren abgeschlossen, das zur Bereitstellung der entsprechenden Steuerwert oder -parameter durchgeführt wurde. Für dieses Vorverfahren ist eine menschliche Mitwirkung und insbesondere die Mitwirkung eines Arztes oder Chirurgen nicht erforderlich. Das Verfahren wird vom Steuergerät 12 ohne Tätigkeit eines Mediziners ausgeführt. Dessen Anwesenheit ist erst für den nachgelagerten chirurgischen Eingriff erforderlich.

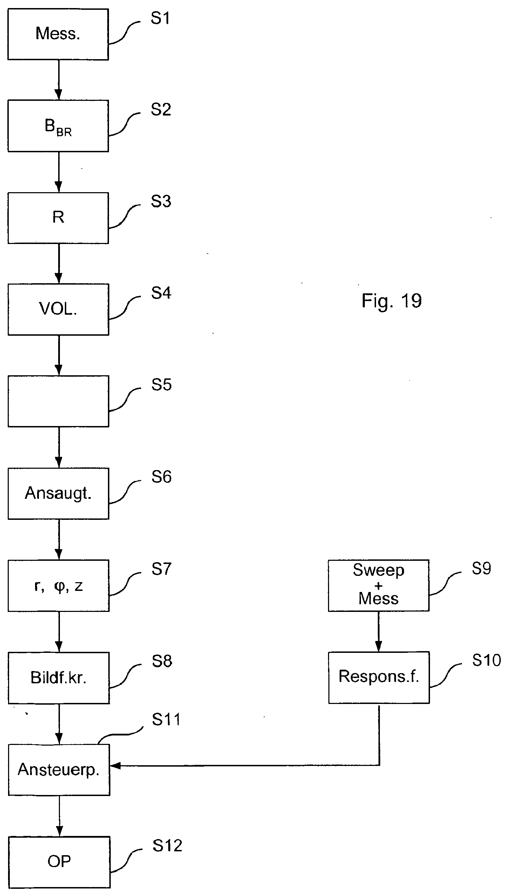

Der Ablauf des Verfahrens zur Vorbereitung des Gerätes 1 auf den Einsatz bei einer augenchirurgischen Fehlsichtigkeitsoperation ist schematisch in Figur 19 zusammengefaßt. In einem Schritt S1 erfolgt die Vermessung des Auges 3. Dabei werden für die beim Patienten 4 vorliegenden Fehlsichtigkeit Korrekturparameter erhalten, wie sie z.B. für herkömmliche Brillen üblich sind. Die in Schritt S2 aufgestellten Parameter werden dann in einem Schritt S3 dazu verwendet, die zur Korrektur nötige neue Krümmung der Hornhaut 5 zu bestimmen. Ist diese Berechnung in Schritt S3 abgeschlossen, wird das Volumen in S4 bestimmt, das aus der Hornhaut entnommen werden muß. Dies geschieht üblicherweise unter Bestimmung der Lentikel-Fläche 20 sowie der Flap-Fläche 19 in einem Schritt S5. Hat man die entsprechenden Funktionsbeschreibungen dieser Flächen gewonnen, wird in Schritt S6 die Ansaugtransformation, die sich beim Ansaugen des Auges an das Kontaktglas auswirkt, berücksichtigt.

Als nächstes werden die Koordinaten der Bahnkurven ermittelt, aus denen die Schnittflächen aufgebaut werden. Dies ist schematisch in Schritt S7 durch die Parameter r, $\varphi$, z angedeutet. Am Ende des Schrittes S7 liegt ein Punkt-Muster mit den Koordinaten der Spots, auf die ein Laserstrahlungspuls einwirken soll. Die Dichte der Zielpunkte kann dabei bereits zur Vereinfachung des rechnerischen Aufwandes reduziert sein, indem nicht für jeden mit Laserstrahlung beaufschlagten Spot eine Stützstelle bei der Ansteuerung der Scanner angegeben wird.

Nachfolgend wird die derart erhaltene Koordinatenmenge in Schritt S8 zur Berücksichtigung des Fokuslagenfehlers nochmals transformiert. In einem Schritt S11 werden dann die eigentlichen Ansteuerparameter ermittelt, wobei eine Response-Funktion eingeht, in einem Schritt S10 aus einer zuvorigen Messung (Schritt S9) des Amplituden- und Frequenzverhaltens der Scanner erhalten wurde.

Mit den derart ermittelten Ansteuerparametern wird dann in Schritt S12 die eigentliche Operation durchgeführt, bei der nun vorzugsweise zwischen den einzelnen Stützstellen, die bei der Ansteuerung des Scanners zugrunde liegen, zusätzliche Spots mit Laserstrahlungspulsen beaufschlagt werden.

**Patentansprüche**

1. Behandlungsvorrichtung zur operativen Fehlsichtigkeitskorrektur eines Auges (3) eines Patienten (4), die eine Steuereinrichtung (12) sowie eine von dieser gesteuerte Lasereinrichtung (L) aufweist, welche einen Laserstrahlung (2) abgebenden Laser (8) und eine diesem nachgeordnete dreidimensionale Fokuslagenverstelleinrichtung (9, 11) aufweist, zur Fokussierung der Laserstrahlung (2) in einen Fokus (7), der in der Hornhaut (5) des Auges (3) liegt, wobei die Lasereinrichtung (L) zur Isolierung eines in der Hornhaut (5) liegenden Volumens (18) durch Einstrahlen der Laserstrahlung (2) zur Trennung von Hornhaut-Gewebe eingerichtet ist, die ferner ein Kontaktglas (25) vorgesehen ist, das zur Fixierung des Auges (3) und zur Verformung einer Vorderfläche (15) der Hornhaut (5) eingerichtet und der Fokuslagenverstelleinrichtung (9, 11) nachgeordnet ist, wobei die Steuereinrichtung (P, 12) in der Hornhaut (5) liegende Zielpunkte (28) festlegt und der Lasereinrichtung (L) vorgibt, um diese hinsichtlich der Fokuslagen der Laserstrahlung (2) in der Hornhaut (5) anzusteuern, und bei der Festlegung der Zielpunkte (28) eine Verformung der Hornhaut (5) des Auges (3) berücksichtigt, die während des Einstrahlens der gepulsten Laserstrahlung (2) durch das Kontaktglas (25) auftritt,

**dadurch gekennzeichnet, daß**

die Steuereinrichtung (P, 12) bei der Vorgabe der Zielpunkte (28) Fokuslagenfehler, die von der Fokuslagenverstelleinrichtung (9, 11) herrühren und beim Fokussieren der Laserstrahlung (2) zu einer Abweichung zwischen vorgegebenen Zielpunkten (28) und tatsächlicher Lage des Fokus (7) führten, durch einen Vorhalt (K) ausgleicht, wobei der Vorhalt (K) von der Lage des jeweiligen Zielpunktes (28) abhängt.

2. Behandlungsvorrichtung nach Anspruch 1, **dadurch gekennzeichnet, daß** die Steuereinrichtung (P, 12) zur Ermittlung des Vorhaltes auf eine Korrekturtabelle (K) zugreift, die den Fokuslagenfehler abhängig von der Lage des jeweiligen Zielpunktes (28) angibt.

3. Behandlungsvorrichtung nach Anspruch 2, **dadurch gekennzeichnet, daß** jeder Vorhalt parallel zur optischen Achse (OA) eine Verschiebung des jeweiligen Zielpunktes (28) bewirkt und die Korrekturtabelle (K) in Zylinderkoordinaten (r, φ, z) nur von axialer und radialer Koordinate (r, z) abhängt.

4. Behandlungsvorrichtung nach einem der obigen Ansprüche, **dadurch gekennzeichnet, daß** die Steuereinrichtung (P, 12) eine Bahn (24) festlegt, entlang der der Fokus (7) der Laserstrahlung zu verstellen ist, auf der Bahn (24) liegende Punkte (6) als Zielpunkte (28) auswählt und diese dann mit dem Vorhalt hinsichtlich des Fokuslagenfehlers korrigiert.

5. Behandlungsvorrichtung nach Anspruch 4, **dadurch gekennzeichnet, daß** die Steuereinrichtung (P, 12) die Bahn (24) durch eine oder mehrere Funktionsgleichung(en) festlegt und die auf der Bahn (24) liegenden Punkte (6) durch Auswertung der eine oder mehrere Funktionsgleichung(en) als Zielpunkte (28) auswählt, und somit die Zielpunkte (28) als Steuerdaten ermittelt.

6. Behandlungsvorrichtung nach Anspruch 4 oder 5, **dadurch gekennzeichnet, daß** die Steuereinrichtung (P, 12) bei der Festlegung der Bahn (24) die Verformung der Hornhaut (5) des Auges (3) berücksichtigt.

7. Behandlungsvorrichtung nach Anspruch 6, **dadurch gekennzeichnet, daß** die Steuereinrichtung (P, 12) zuerst eine Bahn (24) festlegt, die auf das unverformte Auge bezogen ist, und diese dann zum Ausgleichen der Verformung der Hornhaut (5) des Auges (3) modifiziert.

8. Verfahren zum Erzeugen von Steuerdaten für eine Lasereinrichtung (L), wobei

- die Steuerdaten vorgesehen sind für eine Behandlungsvorrichtung (1) zur operativen Fehlsichtigkeitskorrektur eines Auges (3) eines Patienten (4), welche aufweist: einen Laserstrahlung (2) abgebenden Laser (8) und eine diesem nachgeordnete, optische dreidimensionale Fokuslagenverstelleinrichtung (9, 11), welche die Laserstrahlung (2) in einen Fokus (7), der in der Hornhaut (5) des Auges (3) liegt, fokussiert, wobei die Lasereinrichtung (L) zur Isolierung eines in der Hornhaut (5) liegenden Volumens (18) durch Einstrahlen fokussierter Laserstrahlung (2) Hornhaut-Gewebe trennt, wobei ein Kontaktglas (25), das Auge (3) fixiert, das eine Vorderfläche (15) der Hornhaut (5) verformt und der Fokuslagenverstelleinrichtung (9, 11) nachgeordnet ist, und

- die Steuerdaten im Betrieb der Behandlungsvorrichtung (1) der Lasereinrichtung (L) Zielpunkte (28) für die fokussierte Laserstrahlung (2) vorgeben, die in der Hornhaut (5) liegen,

- beim Erzeugen der Steuerdaten eine Verformung der Hornhaut (5) des Auges berücksichtigt wird, die während des Einstrahlens der Laserstrahlung (2) durch das Kontaktglas (25) auftritt, und

**dadurch gekennzeichnet, daß**

- bei der Ermittlung der Zielpunkte (28) Fokuslagenfehler, die von der optischen Fokuslagenverstelleinrichtung (9, 11) herrühren und beim Fokussieren der Laserstrahlung (2) zu einer Abweichung zwischen vorgegebener und tatsächlicher Lage der Zielpunkte (28) führten, durch einen Vorhalt (K) ausgeglichen werden, wobei der Vorhalt (K) von der Lage des jeweiligen Zielpunktes (28) abhängt.

9. Verfahren nach Anspruch 8, **dadurch gekennzeichnet, daß** zur Ermittlung des Vorhaltes eine Korrekturtabelle oder -funktion (K) verwendet wird, die den Fokuslagenfehler abhängig von der Lage des jeweiligen Zielpunktes (28) angibt.

10. Verfahren nach einem der obigen Verfahrensansprüche, **dadurch gekennzeichnet, daß** jeder Vorhalt parallel zur optischen Achse (OA) eine Verschiebung des jeweiligen Zielpunktes (28) bewirkt und die Korrekturtabelle oder -funktion (K), in Zylinderkoordinaten (r, φ, z) nur von axialer und radialer Koordinate (r, z) abhängt.

11. Verfahren nach einem der obigen Verfahrensansprüche, **dadurch gekennzeichnet, daß** eine Bahn (24) festgelegt wird, entlang der der Fokus (7) der Laserstrahlung zu verstellen ist, auf der Bahn (24) liegende Punkte (6) als Zielpunkte (28) auswählt und diese dann mit dem Vorhalt hinsichtlich des Fokuslagenfehlers korrigiert werden.

12. Verfahren nach Anspruch 11, **dadurch gekennzeichnet, daß** die die Bahn (24) durch eine oder mehrere Funktionsgleichung(en) festgelegt wird und die auf der Bahn (24) liegenden Punkte (6) durch Auswertung der eine oder mehrere Funktionsgleichung(en) als Zielpunkte (28) ausgewählt werden, und somit die Zielpunkte (28) als Steuerdaten ermittelt werden.

13. Verfahren nach Anspruch 12, **dadurch gekennzeichnet, daß** bei der Festlegung der Bahn (24) die Verformung der Hornhaut (5) des Auges (3) berücksichtigt wird.

14. Verfahren nach Anspruch 13, **dadurch gekennzeichnet, daß** zuerst eine Bahn (24) festgelegt wird, die auf das unverformte Auge bezogen ist, und diese dann zum Ausgleichen der Verformung der Hornhaut (5) des Auges (3) modifiziert wird.

### Claims

1. A treatment apparatus for surgical correction of eyesight defects in an eye (3) of a patient (4), said apparatus comprising a control device (12) and a laser device (L) which is controlled by the control device (12), and comprises a laser (8), generating laser radiation (2) and feeding a three-dimensional focus adjustment device (9,11) for focussing the laser radiation (2)
into a focus (7) located in the cornea (5) of an eye (3), wherein the laser device (L) is adapted to separate corneal tissue by irradiation of laser radiation (2) to isolate a volume (18) located in the cornea
wherein a contact glass (25) is provided which is adapted for fixation of the eye (3) and deformation of a front surface (15)
of the cornea (5) and which follows
the focus adjustment device (9,11)
wherein
the control device (P, 12) defines target points (28) located in the cornea (5), which are then specified to the laser device (L) in order to control said laser device (L) in terms of focus positions of the laser radiation (2) in the cornea, and
considers a deformation of the cornea (5) of the eye (3) that appears during irradiation of the pulsed laser radiation (2)through the contact glass (25) when defining the target points (28),
**characterized in that**
the control device (P, 12), when specifying the target points (28), compensates for errors in the focus position caused by the focus adjustment device (9, 11), which errors would lead to a deviation between the defined position and the specified position of the focus (7) when focusing the laser radiation (2), by a difference (K) that depends on the position of the respective target point (28).

2. The treatment apparatus as claimed in claim 1, **characterized in that**, in order to determine the difference, the control device (P, 12) accesses a correction table (K) that indicates the focus position error as a function of the respective target point (28).

3. The treatment apparatus as claimed in claim 2, **characterized in that** each difference causes shifting of the respective target point (28) parallel to the optical axis (OA), and the correction table (K) in cylindrical coordinates (r, φ, z),

depends only on the axial and radial coordinates (r, z).

4. The treatment apparatus as claimed in any one of the above claims, **characterized in that** the control device (P, 12) defines a path (24) along with the focus (7) of the laser radiation is to be shifted, selected points (6) located on the path (24) as target points (28), and the corrects them by the difference with respect to the focus position error.

5. The treatment apparatus as claimed in claim 4, **characterized in that** the control device (P, 12) defines the path (24) by one or more functional equation(s) and selects the points (6) located on the path (24) as target points (28) by evaluating said one or more functional equation(s), thus determining the target points (28) as control data.

6. The treatment apparatus as claimed in claim 4 or 5, **characterized in that** the control device (P, 12), when defining the path (24), considers a deformation of the cornea (5) of an eye.

7. The treatment apparatus as claimed in claim 6, **characterized in that** the control device (P, 12) first defines a path (24) for the undeformed eye, and then modifies said path (24) so as to compensate for the deformation of the cornea (5) of the eye (3).

8. A method for generating control data for a laser device (L), wherein

   - the control data are provided for a treatment apparatus (1) for surgical correction of eyesight defects in an eye (3) of a patient (4), the treatment apparatus comprising: a laser (8) emitting laser radiation (2), an optical three-dimensional focus adjustment device (9,11) fed by the laser, which device focuses the laser radiation (2) into a focus (7) located in the cornea (5) of an eye (3), wherein the laser device (L) is adapted to separate corneal tissue by irradiation of focused laser radiation (2) to isolate a volume (18) located in the cornea, wherein a contact glass (25) fixates the eye (3), which contact glass deforms a front surface (15) of the cornea (5) and follows the focus adjustment device (9, 11) and
   - the control data specify to the laser device (L) target points (28) located in the cornea (5) for the focused laser radiation (2) when the treatment apparatus (1) is in operating,
   - when generating control data, a deformation of the cornea (5) of the eye which occurs during irradiation of the laser radiation (2)through the contact glass (25) is considered,

   **characterized in that**,
   when determining the target points (28), focus position errors, resulting from the focus adjustment device (9, 11) which would lead to a deviation between the defined and the actual position of the target points (28) when focusing the laser radiation (2), are compensated for by a difference (K) that depends on the position of the respective target point (28).

9. The method as claimed in claim 8, **characterized in that** a correction table or correction function (K) is used to determine the difference, said table or function indicating the focus position error as a function of the position of the respective target point (28).

10. The method as claimed in any one of the above method claims, **characterized in that** each difference causes a shift of the respective target point (28) parallel to the optical axis (OA), and the correction table or correction function (K), in cylindrical coordinates ($r$, $\varphi$, $z$), depends only on axial and radial coordinates (r, z).

11. The method as claimed in any one of the above method claims, **characterized in that** a path (24) is defined along which the focus (7) of the laser radiation is to be shifted, points (6) located on the path (24) are selected as target points (28) and are then corrected by the difference with respect to the focus position error.

12. The method as claimed in claim 11, **characterized in that** that the path (24) is defined by one or more functional equation(s) and the points (6) located on the path (24) are selected as target points (28) by evaluating said one or more functional equations(s), thus determining the target points (28) as control data.

13. The method as claimed in claim 12, **characterized in that**, when defining the path (24), the deformation of the cornea (5) of an eye (3) is taken into consideration.

14. The method as claimed in claim 13, **characterized in that** a path (24) is first defined for the undeformed eye and is then modified so as to compensate for the deformation of the cornea (5) of an eye (3).

**Revendications**

1. Dispositif de traitement pour la correction opérationnelle d'une déficience visuelle d'un oeil (3) d'un patient (4), qui comprend un dispositif de commande (12) ainsi qu'un dispositif laser (L) commandé par celui-ci, qui comprend un laser (8) générant un rayon laser (2) et un dispositif de réglage de la distance focale (9, 11) tridimensionnelle disposée en aval de celui-ci pour la focalisation du rayon laser (2) au niveau d'un foyer (7), qui se trouve dans la cornée (5) de l'oeil (3), le dispositif laser (L) étant conçu pour l'isolation d'un volume (18) se trouvant dans la cornée (5) par l'action du rayon laser (2) pour la séparation de la cornée et des tissus, un verre de contact (25) étant en outre prévu, qui est conçu pour la fixation de l'oeil (3) et pour la déformation d'une surface avant (15) de la cornée (5) et qui est disposé en aval du dispositif de réglage de la distance focale (9, 11), le dispositif de commande (P, 12) définissant des points cibles (28) se trouvant dans la cornée (5) et les imposant au dispositif laser (L) afin de commander celui-ci en fonction des distances focales du rayon laser (2) dans la cornée (5), et, lors de la définition des points cibles (28), une déformation de la cornée (5) de l'oeil (3), qui survient pendant la projection du rayon laser (2) pulsé à travers le verre de contact (25),
   **caractérisé en ce que**
   le dispositif de commande (P, 12) compense, avec anticipation (K), lors de la définition des points cibles (28), des erreurs de distances focales, qui proviennent du dispositif de réglage de distance focale (9, 11) et mènent, lors de la focalisation du rayon laser (2), à un écart entre des points cibles (28) prédéterminées et la position réelle du foyer (7), l'anticipation (K) dépendant de la position du point cible (28) correspondant.

2. Dispositif de traitement selon la revendication 1, **caractérisé en ce que** le dispositif de commande (P, 12) accède, pour la détermination de l'anticipation, à un tableau de correction (K), qui donne l'erreur de distance focale en fonction du point cible (28) correspondant.

3. Dispositif de traitement selon la revendication 2, **caractérisé en ce que** chaque anticipation provoque, parallèlement à l'axe optique (OA), un décalage du point cible (28) correspondant et le tableau de correction (K) en coordonnées cylindriques (r, $\varphi$, z) dépend uniquement des coordonnées axiales et radiales (r, z).

4. Dispositif de traitement selon l'une des revendications précédentes, **caractérisé en ce que** le dispositif de commande (P, 12) définit une trajectoire (24) le long de laquelle le foyer (7) du rayon laser doit être déplacé, sélectionne des points (6) se trouvant sur la trajectoire (24) en tant que points cibles (28) et corrige ensuite ceux-ci avec l'anticipation en ce qui concerne l'erreur de distance focale.

5. Dispositif de traitement selon la revendication 4, **caractérisé en ce que** le dispositif de commande (P, 12) définit la trajectoire (24) à l'aide d'une ou plusieurs équations fonctionnelles et sélectionne des points (6) sur la trajectoire (24), par une analyse de l'une ou plusieurs équations fonctionnelle en tant que points cibles (28) et définit ainsi les points cibles (28) en tant que données de commande.

6. Dispositif de traitement selon la revendication 4 ou 5, **caractérisé en ce que** le dispositif de commande (P, 12) prend en compte, lors de la définition de la trajectoire (24), la déformation de la cornée (5) de l'oeil (3).

7. Dispositif de traitement selon la revendication 6, **caractérisé en ce que** le dispositif de commande (P, 12) définit d'abord une trajectoire (24) relative à l'oeil non déformé et modifie ensuite celle-ci pour compenser la déformation de la cornée (5) de l'oeil (3).

8. Procédé de génération de données de commande pour un dispositif laser (L),

   - les données de commande étant conçues pour un dispositif de traitement (1) pour la correction opérationnelle d'une déficience visuelle d'un oeil (3) d'un patient (4), qui comprend : un laser (8) émettant un rayon laser (2) et un dispositif de réglage de distance focale tridimensionnelle optique (9, 11), disposée en aval de celui-ci, qui focalise le rayon laser (2) au niveau d'un foyer (7) qui se trouve dans le volume (18) se trouvant dans la cornée (5) de l'oeil (3), le dispositif laser (L), pour l'isolation d'un volume (18) se trouvant dans la cornée (5), séparer la cornée et les tissus par la projection d'un rayon laser (2) focalisé, un verre de contact (25) fixant l'oeil (3), qui déforme une surface avant (15) de la cornée (5) et est disposé en aval du dispositif de réglage de distance focale (9, 11) et
   - les données de commande définissant, lors du fonctionnement du dispositif de traitement (1) du dispositif laser (L), des points cibles (28) pour le rayon laser focalisé (2), qui se trouvent dans la cornée (5),
   - lors de la génération des données de commande, une déformation de la cornée (5) de l'oeil, qui survient pendant la projection du rayon laser (2) à travers le verre de contact (25), étant prise en compte et

**caractérisé en ce que**

- lors de la détermination des points cibles (28), les erreurs de distance focale, qui proviennent du dispositif de réglage de distance focale optique (9, 11) et ont mené, lors de la focalisation du rayon laser (2), à un écart entre la position prédéterminée et la position réelle des points cibles (28), sont compensées grâce à une anticipation (K), l'anticipation (K) dépendant de la position du point cible (28) correspondant.

9. Procédé selon la revendication 8, **caractérisé en ce que**, pour la détermination de l'anticipation, un tableau ou une fonction de correction (K) est utilisée, qui donne l'erreur de distance focale en fonction de la position du point cible (28) correspondant.

10. Procédé selon l'une des revendications précédentes, **caractérisé en ce que** chaque anticipation provoque, parallèlement à l'axe optique (OA), un décalage du point cible (28) correspondant et le tableau ou la fonction de correction (K) en coordonnées cylindriques (r, (p, z) dépend uniquement des coordonnées axiales et radiales (r, z).

11. Procédé selon l'une des revendications précédentes, **caractérisé en ce qu'**une trajectoire (24) est définie, le long de laquelle le foyer (7) du rayon laser doit être réglé, sélectionne des points (6) se trouvant sur la trajectoire (24) en tant que points cibles (28) et ceux-ci sont ensuite corrigés avec l'anticipation en ce qui concerne l'erreur de distance focale.

12. Procédé selon la revendication 11, **caractérisé en ce que** la trajectoire (24) est définie par une ou plusieurs équations fonctionnelles et les points (6) se trouvant sur la trajectoire (24) sont sélectionnés par analyse de l'une ou plusieurs équations fonctionnelles en tant que points cibles (28) et les points cibles (28) sont ainsi définis en tant que données de commande.

13. Procédé selon la revendication 12, **caractérisé en ce que**, lors de la définition de la trajectoire (24), la déformation de la cornée (5) de l'oeil (3) est prise en compte.

14. Procédé selon la revendication 13, **caractérisé en ce qu'**une trajectoire (24) est d'abord définie, qui est relative à l'oeil non déformé et celle-ci est ensuite modifiées pour compenser la déformation de la cornée (5) de l'oeil (3).

FIG 1

FIG 2

Fig. 5

Fig. 6

Fig. 1a

## FIG 3

Fig. 7

Fig. 4

a)

b)

c)

FIG 8

FIG 9

Fig 10

Fig. 11

**FIG 12**

$3 \downarrow$

*Fig. 13*

$25 \downarrow$

*Fig. 14*

Fig. 15

Fig. 18

Fig. 16

Fig. 17

Fig. 19

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5984916 A
- US 20040070761 A1
- WO 2005011547 A
- EP 1159986 A1
- US 5549632 A
- DE 69500997 T2
- WO 2004032810 A2
- WO 2005011546 A
- WO 2005011545 A
- WO 2005048895 A
- WO 2003002008 A
- WO 2005011547 A1